# EUROPEAN PATENT SPECIFICATION

(11) **EP 0 819 758 B1**
(45) Date of publication and mention of the grant of the patent: **20.09.2006**
(21) Application number: 97112154.6
(22) Date of filing: 16.07.1997
(51) Int. Cl.: C12N 15/12, C12N 15/24, A61K 48/00

(54) **Cationic vehicle: DNA complexes and their use in gene therapy**
Kationisches Vehikel: DNS Komplexe und ihre Verwendung in Gentherapie
Complexes véhicule cationique: ADN et leur utilisation dans la thérapie génique

(30) Priority: 16.07.1996 US 680845
(43) Date of publication of application: 21.01.1998
(60) Divisional application: 02025984.2
(73) Proprietor: Mixson, Archibald James, Rockville, MD 21201 (US)
(72) Inventor: Mixson, Archibald James, Rockville, MD 21201 (US)
(74) Representative: Sieckmann, Ralf

(56) References cited:
- WO-A-92/02240
- WO-A-95/30330
- WO-A-95/31559
- WO-A-96/21416
- WEINSTAT-SASLOW ET AL: "TRANFECTION OF THROMBOSPONDIN 1 COMPLEMENTARY DNA INTO A HUMAN BREAST CARCINOMA CELL LINE REDUCES PRIMARY TUMOR GROWTH, METASTATIC POTENTIAL, AND ANGIOGENESIS" CANCER RESEARCH, vol. 54, 1994, pages 6504-6511, XP002048545
- LASIC D.D. ET AL: 'Liposomes revisited' SCIENCE vol. 267, 1995, pages 1275 - 1276
- ZHU N. ET AL: 'Systemic gene expression after intravenous DNA delivery into adult mice' SCIENCE vol. 261, 1993, pages 209 - 211, XP001068973
- MARSHALL E.: 'Gene therapy's growing pains' SCIENCE vol. 269, 1995, pages 1050 - 1055, XP000676418
- LESON-WOOD L.A.: 'systemic gene therapy with p53 reduces growth and metastases of a malignant human breast cancer in nude mice' HUMAN GENE THERAPY vol. 6, 1995, pages 395 - 405, XP002070391
- VILE R.G.: 'Gene therapy' CURRENT BIOLOGY vol. 8, no. 3, 1998, pages R73 - R75
- FRIEDMANN T.: 'Human gene therapy - an immature genie, but certainly out of the bottle' NATURE MEDICINE vol. 2, no. 2, 1996, pages 144 - 147
- SONG LI; LEAF HUANG: 'Lipidic supramolecular assemblies for gene transfer' JOURNAL OF LIPOSOME RESEARCH vol. 6, no. 3, 1996, pages 589 - 608

## Description

### FIELD OF THE INVENTION

The present invention relates to cationic vehicles:DNA complexes (i.e. cationic liposome:DNA complexes, cationic polymer:DNA complexes) as defined in the claims.

### BACKGROUND OF THE INVENTION

### I. Gene Therapy

Development of gene therapy techniques is approaching clinical realization for the treatment of neoplastic and metabolic diseases. The main obstacle in the treatment of malignant diseases, however, remains in the vector delivery system of the transgene to a distant target tissue.

Vectors carrying genes are commonly divided into viral and non-viral vector categories. Unfortunately, all vectors described to date have significant limitations. For example, replication-deficient retroviral vectors can efficiently transfect dividing cells. Local intratumoral injection of retroviruses that contain a thymidine kinase transgene has been used successfully to affect regression of gliomas (Culver et al, *Science,* 256:1550-1552 (1992)). However, retroviruses have the potential to cause insertional mutagenesis. As a result, their use has been limited to either direct injection of tumors or to *ex vivo* gene transfer trials. Unlike retroviral vectors, adenoviral vectors can also transfect non-dividing cells, and their ability to cause insertional mutagenesis is greatly reduced. However, they have the undesirable potential to activate the immune system in humans (Crystal, *Science*, 270:404-410, (1995). Attempts are underway to minimize the immunogenicity of the adenoviral vectors, but the potential toxicity of viral vectors will most likely limit their use for systemic delivery of genes in the near future.

Non-viral vectors of DNA include liposomes, peptides, proteins and polymers (Ledley, *Current Opinion in Biotechnology,* 5:626-63.6 (1994)). Of these, liposomes are the most commonly used non-viral vectors of DNA. The major advantage of liposomes over retroviruses is that DNA is not incorporated into the genome, and unlike adenoviral vectors, they are not immunogenic. However, the major limitation of liposomes is that they are not as efficient as viral vectors in transfecting many cell types. Until recently, their medical utility was limited by their rapid uptake by phagocytic cells. Interest in liposomes as a vector was rejuvenated by two technological advances that have produced a renaissance in the field. First, stearically stabilized (Stealth) liposomes represent a significant breakthrough in that they are non-reactive, and are not readily taken up by the reticuloendothelial system (RES). Stealth liposomes are composed of lipids rich in oxygen in their head group (ethylene glycol or glycolipids) which provide a stearic barrier outside of the membrane. As a result, Stealth liposomes remain in the blood for up to 100 times longer than conventional liposomes, and can thus increase pharmacological efficacy (Papahajopoulos, *In*: *Stealth liposomes,* Ed., Lasic et al, CRC Press' (1995); and Lasic et al, *Science,* 267:1275-76 (1995)). However, Stealth liposomes are still not particularly efficient in transfection of cells or as vectors for DNA.

The second significant advance in liposome technology has been the use of cationic liposomes complexed to negatively-charged DNA. Cationic liposomes can condense DNA, and increase transfection yields several orders of magnitude. In the cationic liposome:DNA complex, the nucleic acids or oligonucleotides are not encapsulated, but are simply complexed with small unilamellar vesicles by electrostatic interactions. The exact nature of the cationic liposome:DNA complex is not clear, but intricate topological rearrangements of the cationic liposome:DNA complex occur, including DNA condensation, liposome aggregation, and fusion. This supramolecular complex can be added to cells *in vitro,* injected parenterally, or aerosolized for pulmonary applications (Lasic et al, *Science*, 267:1275-1276 (1995)). Further, the intravenous injection into mice of high concentrations of the CAT gene (100 µg or greater) complexed with cationic liposomes has been found to result in 40% transfection efficiency of well vascularized tissues, such as the spleen (Zhu et al, *Science*, 261:209-211 (1993)). However, a major challenge of gene therapy remains the systemic delivery of transgenes to the tumor or peritumoral area that will effectively decrease the size of primary tumors and their metastases. This is because unlike the spleen and bone marrow, which are highly vascular and have a high capacity to filter macromolecules from the blood stream, most organs and tumors do not have this capacity, and the transfection efficiency of these tissues with liposomes is low (Marshall, *Science,* 269:1051-1055 (1995)). In addition, another limitation of cationic liposome: DNA complexes is that their 1/2 life in the blood stream is less than one hour (Allen et al, In: *Liposome Technalogy*-Vol. III, Ed., Gregoriadis G et al, CRC Press (1993)). Sufficient transfection of the target cell by vectors carrying therapeutic genes has thus far been the rate-limiting step in gene therapy.

### II. Tumor Suppressor Genes

Tumor suppressor genes are well-known in the art, and include the p53 gene (Baker et al, *Science*, 249:912-915 (1990)), the p21 gene (El-Deiry et al, *Cell*, 75:817-825 (1993); and Harper et al, *Cell,* 75:805-816 (1993)), and the rb gene (Bookstein et al, *Science*, 247:712-715 (1990)).

Mutations in the tumor suppressor gene p53 are known to occur in over 50% of human tumors, including metastatic breast cancer (Vogelstein, Nature, 348:681-682 (1990)). Breast cancer is one of the leading causes of death in women in North America and Western Europe, affecting nearly 10% of this population living to 80 years of age, and one million new cases are predicted by the end of this decade (Miller et al, *Int. J. Cancer,* 37:173-177 (1986)). Although the molecular basis of multistage carcinogenesis in breast cancer is not well understood, the metastatic potential of breast cancers has been correlated with the presence of point mutations in the p53 gene (Wang et al, *Oncogene,* 8:279-288 (1993)). Various groups have found that reintroduction of the wild-type P53 into a tumor cell has the therapeutic potential to inactivate the proliferative effects of the mutated product (Bookstein et al, Cancer, 71:1179-1186 (1993); Chen et al, *Science,* 250:1576-1580 (1990); and Baker et al, *Science*, 249:912-915 (1990)). For example, *in vitro* transfection and retroviral-mediated transfer of a single copy of the p53 transgene into a variety of tumor cells, including breast cancer cells, was found to result in a decrease in growth rate and/or attenuated tumor development once those transfected cells were implanted into nude mice (Wang et al, Oncogene, 8:279-288 (1993); Baker et al, *Science,* 249:912-915 (1990)); Bookstein et al, Science, 247:712-715 (1990); Cheng et al, *Cancer Res.,* 52:222-226 (1992); Isaacs et al, *Cancer Res*., 51:4716-4720 (1991); Diller et al, *Mol. Cell. Biol.,* 10:5772-5781 (1990); Chen et al, *Oncogene*, 6:1799-1805 (1991); and Zou et al, *Science*, 263:526-529 (1994)). In addition, intratracheal injection of a retrovirus containing the p53 transgene has been shown to significantly inhibit the growth of lung tumors (Fujiwara et al, *J. Natl. Cancer. Inst*., 86:1458-1462 (1994)). Further, systemic intravenous administration of a β-actin promoter-containing vector containing the p53 coding sequence complexed to cationic liposomes has been found to affect the tumor growth of a malignant line of breast cancer cells injected into nude mice (Lesoon-Wood et al, *Proc. Am. Ass. Cancer Res.,* 36:421 (1995); and Lesoon-Wood et al, *Human Gene Ther.,* 6:39-406 (1995)). Of the 15 tumors treated in this study, four of these tumors did not respond to treatment. Because of the unresponsiveness of these tumors, new therapies were sought in the present invention to more effectively decrease the size of these tumors. Based on the in vitro data concerning p53, one might expect that p53 decreases the size of the tumors due to efficient transfection of the tumor. However, less than 5% of the tumor was transfected after three injections of a cationic liposome:marker (CAT) gene. Furthermore, some endothelial cells of the tumor were transfected with this marker gene. Thus, the primary target of cationic liposome:p53 complex may be the vasculature system of the tumor. Given that angiogenesis is critical for the development of any human tumor, as well as for metastastases (Fidler et al, *Cell*, 79:185-188 (1994)), this therapy should be widely applicable to a wide variety of tumors.

p53 coordinates multiple responses to DNA damage. DNA damage results in an increase in the level of the p53 protein. Following DNA damage, an important function of wild-type p53 function is to control the progression of cells from G1 to S phase. Recently, several groups have found that p53 transcriptionally activates a p21 kd protein (also known as WAF1 or Cip1), an inhibitor of cyclin-dependent kinases (CDKs) (E1-Deiry et al, *supra;* and Harper et al, *supra*). Inhibition of CDK activity is thought to block the release of the transcription factor E2F, and related transcription factors from the retinoblastoma protein RB, with consequent failure to activate transcription of genes required for S phase entry (Harper et al, *supra;* and Xiong et al, Nature, 366:701-704 (1993)).

### III. Anti-Angiogenic Proteins

Proteins with anti-angiogenic activities are well-known and include: thrombospondin I (Kosfeld et al, *J. Biol. Chem*., 267:16230-16236 (1993); Tolsma et al, *J. Cell Biol.,* 122:497-511 (1993); and Dameron et al, *Science,* 265:1582-1584 (1995)), IL-12 (Voest et al, *J. Natl. Cancer Inst.,* 87:581-586 (1995)), protamine (Ingber et al, *Nature*, 348:555-557 (1990)), angiostatin (O'Reilly et al, *Cell*, 79:315-328 (1994)), laminin (Sakamoto et al, *Cancer Res.,* 5:903-906 (1991)), and a prolactin fragment (Clapp et al, *Endocrinol*., 133:1292-1299 (1993)). In addition, several anti-angiogenic peptides have been isolated from these proteins (Maione et al, *Science*, 247:77-79 (1990); Woltering et al, *J*. *Surg. Res.,* 50:245-251 (1991); and Eijan et al, *Mol. Biother*., 3:38-40 (1991)).

Thrombospondin I (hereinafter "TSPI") is a large trimeric glycoprotein composed of three identical 180 kd subunits (Lahav et al, *Semin. Thromb. Hemostasis,* 13:352-360 (1987)) linked by disulfide bonds (Lawer et al, *J. Cell Biol.,* 103:1635-1648 (1986); and Lahav et al, *Eur. J. Biochem*., 145:151-156 (1984)). The majority of anti-angiogenic activity is found in the central stalk region of this protein (Tolsma et al, *supra*). There are at least two different structural domains within this central stalk region that inhibit neovascularization (Tolsma et al, *supra*).

Besides TSPI, there are five other proteins (fibronectin, laminin, platelet factor-4, angiostatin, and prolactin fragment) in which peptides have been isolated that inhibit angiogenesis. In addition, analogues of the peptide somatostatin are known to inhibit angiogenesis.

Fibronectin (FN) is a major surface component of many normal cells, as well as a potent dell spreading factor. During transformation, the loss of cellular FN has been observed. Furthermore, the addition of fibronectin to transformed cells restores the normal phenotype. It has been found that either heparin-binding or cell-adhesion fragments from FN can inhibit experimental metastasis, suggesting that cell surface proteolyglycans are important in mediating the adhesion of metastatic tumor cells (McCarthy et al, *J. Natl. Cancer Inst*., 80:108-116 (1988)). It has also been found that FN and one of its peptides inhibits in vivo angiogenesis (Eijan et al, *Mol. Biother*., 3:38-40 (1991)).

Laminin is a major component of the basement membrane, and is known to have several biologically active sites that bind to endothelial and tumor cells. Laminin is a cruciform molecule that is composed of three,chains, an A Chain and two B chains. Several sites in laminin have been identified as cell binding domains. These sites promote cellular activities in vitro, such as cell spreading, migration, and cell differentiation. Two peptides from two sites of the laminin Bl chain are known to inhibit angiogenesis (Grant et al, *Path. Res. Pract.*, 190:854-863 (1994)).

Platelet factor-4 (PF4) is a platelet α-granule protein originally characterized by its high affinity for heparin. The protein is released from platelets during aggregation as a high molecular weight complex of a tetramer of the PF4 polypeptide and chondroitin sulfate, which dissociates at high ionic strength. PF4 has several biological properties including immunosuppression, chemotactic activity for neutrophils and monocytes as well as for fibroblasts, inhibition of bone resorption, and inhibition of angiogenesis. The angiostatic properties of human PF4 are associated with the carboxyl-terminal, heparin binding region of the molecule. A 12 amino acid synthetic peptide derived therefrom has been discovered to have marked angiostatic affects (Maione et al, *Science,* 247:77-79 (1990)).

Although somatostatin is not a protein, it is a naturally-occurring cyclic 14 amino acid peptide whose most-recognized function is the inhibition of growth hormone (GH) secretion. Somatostatin is widely distributed in the brain, in which it fulfills a neuromodulatory role, and in several organs of the gastrointestinal tract, where it can act as a paracrine factor or as a true circulating factor. The role played by the neuropeptide somatostatin, also known as somatotropin release inhibitory factor (SRIF), in human cancer is not well understood. Recent investigations involving somatostatin receptors in normal and neoplastic human tissues suggest that the action is complex, and involves both direct and indirect mechanisms. One of the anti-tumor mechanisms of these synthetic somatostatin analogues may be an anti-angiogenic effect (Woltering et al, *J. Surg. Res.,* 50:245-50 (1990)). In a recent study, the ability of native somatostatin and nine somatostatin analogues to inhibit angiogenesis were evaluated. The most potent somatostatin analogue was found to be approximately twice as potent as the naturally-occurring somatostatin (Barrie et al, *J. Surg. Res.*, 55:446-50 (1993)).

Angiostatin is a 38 kDa polypeptide fragment of plasminogen. Whereas plasminogen has no fibrinogenic activity, angiostatin has marked angiogenic activity (O'Rielly MS, et al Cell, 79:315-28 (1994)). Angiostatin was isolated when it was observed that the primary tumor suppresed metastases. That is, when the primary tumor was removed, the metastases grew. Admininistration of angiostatin blocks neovascularization and growth of metastases.

Finally, a 16kd fragment of prolactin has been found to be angiogenic. Similar to plasminogen, prolactin is not anti-angiogenic but the prolactin fragment is a potent in vivo and in vitro inhibitor of angiogenesis (Clapp C. et al. Endocrinology. 133:1292-1299 (1993).

Despite the evidence that anti-angiogenic peptides are effective anti-tumor agents, as well as the great deal of interest in targeting genes toward the vasculature, there have been no published reports on effective *in vivo* gene therapy regimens with established anti-angiogenic DNA sequences.

There are several reasons why gene therapy utilizing antiangiogenic genes have not been used or why antiangiogenic peptides are effective and the liposome: antiangiogenic gene may not be. First, there are significant physical differences between the liposome: DNA complexes and their peptides. Cationic liposomes have a 1/2 life of less than one hour (Allen TM and Papahajopoulos D, In: Liposome Technology-Vol. III, Ed., Gregoriadis G et al, CRC Press (1993)). whereas the most effective of the antiangiogenic peptides (i.e angiostatin) have a 1/2 life of two days (Folkman J, The John Krantz, Jr Lecture in Pharmacology, UMAB, 4/30/96). Since cationic liposomes form large aggregates when mixed with DNA, the distribution of these complexes is likely to be quited different from the much smaller peptides (need reference). These properties of the liposomes may account for the low transfection efficiency of a tumor. Therefore, it is uncertain as to whether these liposome:DNA complexes will reach their cellular targets.

Furthermore, the exact receptor target or mechanisms of these antiangiogenic peptides are unknown (Tolsma et al, supra). For example, it is unknown whether these receptor targets are intracellular or extracellular. The anti-angiogenic genes that are complexed to liposomes encode their respective proteins inside the cell, and proteins without secretory sequences remain inside the cell. Thus, it is unclear that a intracellular antiangiogenic peptide derived from a systemically transfected gene will reach its cellullar and/or receptor target.

The only transfected antiangiogenic gene that has inhibited tumor growth is full length thrombospondin I. In this study *(Weinstat-Saslow et al, Cancer Research 54, 6504-6511, (1994))* tumor cells that expressed 15 fold higher levels of the thrombospondin I in vitro than baseline cells were implanted into the mice. This transfected full length thrombospondin I was secreted from the tumor cells to inhibit angiogeneis, and effectively reduced the tumor by 60%. Thus, this study determined that transfection of 100% of the tumor cells with a highly expressed secreted antiangiogenic gene was able to reduce tumor size.

### SUMMARY OF THE INVENTION

An object of the present invention is to provide cationic vehicles:DNA complexes, namely liposome complexes containing DNA encoding anti-angiogenic peptides or cationic complexes containing DNA encoding an anti-angiogenic peptides both together with the tumor suppressor protein p53.

Yet another object of the present invention is to provide liposome complexes containing DNA encoding an anti-angiogenic peptide in combination with DNA encoding a tumor suppressor gene.

A further object of the present invention is to provide liposome complexes containing DNA encoding concatamers of the same or different anti-angiogenic peptides.

An additional object of the present invention is to use these complexes for the production of a medicament.

These and other objects of the present invention, which will be apparent from the detailed description of the invention provided hereinafter, have been met in one embodiment, by a cationic liposome:DNA complex comprising DNA encoding an anti-angiogenic peptide and DNA encoding a tumor suppressor protein.

Thus, the invention relates to a cationic liposome:DNA complex comprising DNA encoding an anti-angiogenic peptide said complex additionally comprises DNA encoding a tumor suppressor protein wherein said tumor suppressor protein is p53 and wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

Further, it relates to the use of a complex for the production of a medicament to inhibit tumor angiogenesis as above, wherein said cationic liposome includes preferably 1,2-dioleoyl-sn-glycero-3- ethylphosphocholine, 1,2-dimyristoyl-sn-glycero-3- ethylphosphocholine, and N-[1-(2,3-dioleoyloxy)propyl]- N,N,N- trimethylammonium chloride and may also be comprised of polyethylene glycol preferably a pegylated lipid-1,2 -diacyl-sn-glycero-3-phosphoethanolamine-N-[Poly-(ethylene glycol) 2000 and a targeted ligand preferably the amino acid peptide RGD, ferritin, or antibodies targeted toward to HER2.

Based on the present invention, it is anticipated that one skilled in the gene therapy could utilize other cationic carriers (polylysine, polyhistidine, polycat57, Superfect, and polyethyleneimine) complexed with the antiangiogenic genes to inhibit tumors.

### DETAILED DESCRIPTION OF THE INVENTION

As discussed above, in one embodiment, the above-described objects of the present invention have been met by a non-viral:DNA complex comprising DNA encoding an anti-angiogenic peptide and DNA encoding a tumor suppressor protein.

The particular non-viral carrier (liposomes-neutral or non-cationic, see below), polyethylenimine Polycat57 (Avanti Lipids), polylysine (Sigma), polyhistidine (Sigma), Superfect (Qiagen), are not critical to the present invention although cationic liposomes are preferable carriers. Examples of cationic lipids which can be employed in the present invention

Moreover, the inventon relates to a cationic polymer:DNA complex comprising the DNA encoding an antiangiogenic peptide, wherein said complex additionally comprises DNA encoding a tumor suppressor protein wherein said tumor suppressor protein is p53 wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30 and the invention relates to the use of a cationic polymer:DNA complex above comprising DNA encoding an anti-angiogenic peptide for the production of a medicament for inhibiting tumor growth in a subject which preferably comprises administering the same to a tumor-bearing subject and to use of Claim 11, where said complex additionally comprises DNA encoding a tumor suppressor protein. include 1,2-dioleolyl-sn-glycero-3-ethylphosphocholine (Avanti, Birmingham, AL), 1,2-dimyristoyl-sn-glycero-3-ethylphosphocholine (Avanti, Birmingham, AL), and (2,3-diol-eyloxy)propyl-N,N,N-trimethylammonium chloride (DOTMA) (Syntex Corp., Palo Alto, CA). The cationic lipids are preferably used in a mixture with dioleoylphophatidylethanolamine (DOPE) (Avanti, Bimingham, AL). In the present invention, the amount of cationic lipid present in the mixture is generally in the range of from 100% to 40% (w/w), preferably about 50% (w/w); and the amount of DOPE present in the mixture is generally in the range of from 0% to 60% (w/w), preferably about 50% (w/w); and the amount of pegylated lipid (1,2 -diacyl-sn-glycero-3-phosphoethanolamine-N-[Poly(ethylene glycol) 2000] present in the mixture is generally in the range of from 0% to 10% (w/w), preferably about 1% (w/w).

The particular ligand will be dependendent on the the tumor/peritumoral targeted. Examples of targets on tumors include Her2 (breast), CEA (colon), ferritin receptor (breast, lung, and ovary), and the tumor vasculature (αvB3 integrins or tissue factor). Antibodies directed toward Her2, CEA, and the tumor's vasculature will be coupled to 1% of the pegylated lipid hydroxyl group of the pegylated lipid with a water soluble carbodiimide (1-ethyl-3 (3-dimethylamino-propyl) carbodiimide), and purified over a sepharose CL-6B column. Similarly, ligands to the tumor (ferritin) and/or the vasculature (the peptide, RGD) are covalently attached to the hydroxyl the pegylated lipids.

The particular tumor suppressor gene employed is the p53 gene. The p53 gene is the tumor suppressor gene employed in the present invention.

Examples of anti-angiogenic peptides include a fragment of thrombospondin I (TSPf) having the following amino acid sequence (the amino acid sequences that are known to be anti-angiogenic are underlined):
MTEENKELANELRRPPLCYHNGVQYRNNEEWTVDSCTECHCQNSVTICKKVSCP IMPCSNATVPDGECCPRCWPSDSADDGWSPWSEWTSCSTSCGNGIQQRGRSCDS LNNRCEGSSVQTRTCHIQECDKRFKQDGGWSHWSPWSSCSVTCGDGVITRITLC NSPSPQMNGKPCEGEARETKACKKDACPINGGWGPWSPWDICSVTCGGGVQKRS RL (SEQ ID NO:1), which is encoded by the following DNA sequence (nucleotides 1013-1650 of the TSPI gene; the underlined sequences encode the anti-angiogenic peptides; the bold TAA is the stop codon):
a concatamer of TSPf having the following amino acid sequence (the intervening sequence is underlined): MTEENKEIANELPRPPLCYHNGVQYRNNEEWTVDSCTECHCQNSVTICKKVSCP IMPCSNATVPDGECCPRCWPSDSADDGWSPWSEWTSCSTSCGNGIQQRGRSCDS LNNRCEGSSVQTRTCHIQECDKRFKQDGGWSHWSPWSSCSVTCGDGVITRITLC NSPSPQMNGKPCEGFARETKACKKDACPINGGWGPWSPWDICSVTCGGGVQKRS RLCVHSRMTEENKELANELRRPPLCYHNGVQYRNNEEWTVDSCTECHCQNSVTI CKKVSCPIMPCSNATVPDGECCPRCWPSDSADDGWSPWSEWTSCSTSCGNGIQQ RGRSCDSLNNRCEGSSVQTRTCHIQECDKRFKQDGGWSHWSPWSSCSVTCGDGV ITRITLCNSPSPQMNGKPCEGEARETKACKKDACPINGGWGPWSPWDICSVTCG GGVQKRSRL (SEQ ID NO:3), which is encoded by the following DNA sequence (the intervening sequence is underlined) :
laminin peptide having the following amino acid sequence: MYIGSR (SEQ ID NO:5), which is encoded by the following DNA sequence (the *Sal*I sites are underlined, and the stop codon is in bold): GTCGACATGTATATTGGTTCTCGTT**AA**GTCGAC (SEQ ID NO:6.);
a concatamer of the laminin sequence having the following amino acid sequence (the intervening sequences are underlined):
   MYIGSRGKSYIGSRGKSYIGSRGKS (SEQ ID NO:7), which is encoded by the following DNA sequence (the *Sal*I sites are underlined, and the intervening sequences are in bold):
   GTCGACATGTATATTGGTTCTCGTGG**TAAAAGA**TATATTGGTTCTCGT**GGTAAA AGA**TATATTGGTTCTCGT**GGTAAAAGA**TAAGTCGACC (SEQ ID NO:8);
   a peptide from platelet factor-4 having the following amino acid sequence:
      MLYKKIIKKLLES (SEQ ID NO:9), which is encoded by the following DNA sequence (the *Sal*I sites are underlined) :
         GTCGACATGCTTTATAAGAAGATCATCAAGAAGCTTCTTGAGAGTTAAGTCGAC (SEQ ID NO:10);
a concatamer of the platelet factor-4 peptide having the following amino acid sequence (the intervening sequences are underlined):
   MLYKKIIKKLLESGKSLYKKIIKKLLESGKSLYKKIIKKLLESGKS (SEQ ID NO:11), which is encoded by the following DNA sequence (the *Sal*I sites are underlined, and the intervening sequences are in bold):
   somatostatin inhibitor having the following amino acid sequence: MFCYWKVCW (SEQ ID NO:13), which is encoded by the following DNA sequence (the *Sal*I sites are underlined):
      GTCGACATGTTCTTGTATTGCAAGGGATTGTGGTAAGTCGAC (SEQ ID NO:14);
a concatamer of somatostatin inhibitor having the following amino acid sequence (the intervening sequences are underlined):
   MFCYWKVCWGKSFCYWKVCWGKSFCYWKVCWGKS (SEQ ID NO:15), which is encoded by the following DNA sequence (the *Sal*I sites are underlined, and the intervening sequences are in bold):
fibronectin inhibitor having the following amino acid sequence: MGRGD (SEQ ID NO:17), which is encoded by the following DNA sequence (the *Sal*I sites are underlined) :
   GTCGACATGTCTTTGTCTTGGAAGACTTTGACTTAAGTCGAC (SEQ ID NO:18);
a concatamer of fibronectin inhibitor having the following amino acid sequence (the intervening sequences are underlined):
   MGRGDGKSGRGDGKSGRGDGKS (SEQ ID NO:19); which is encoded by the following DNA sequence (the *Sal*I sites are underlined, and the intervening sequences are in bold):
      GTCGACATGGGTCGTGGTGAT**GGTAAAAGA**GGTCGTGGTGAT**GGTAAAAGA** GGTCGTGGTGATGGTAAAAGATAAGTCGAC (SEQ ID NO:20);
      angiostatin having the following amino acid sequence:
         MVYLSECKTGIGNGYRGTMSRTKSGVACQKWGATFPHVPXYSPSTHPNEGLEEN YCRNPDNDEQGPWCYTTDPDKRYDYCNIPECEEECMYCSGEKYEGKISKTMSG LDCQAWDSQSPHAHGYIPAKFPSKNLKMNYCHNPDGEPRPWCFTTDPTKRWEYC DIPRCTTPPPPPSPTYQCLKGRGENYRGTVSVTVSGKTCQRWSEQTPHRHNRTP ENFPCKNLEENYCRNPDGETAPWCYTTDSQLRWEYCEIPSCESSASPDQSDSSV PPEEQTPWQECYQSDGQSYRGTSSTTITGKKCQSEQTPHR (SEQ ID NO:21), which is encoded by the following DNA sequence (the *Sal*I sites are underlined):
            GTCGACATGGTGTATCTGTCAGAATGTAAGACCGGCATCGGCAACGGC TACAGAGGAACCATGTCCAGGACAAAGAGTGGTGTTGCCTGTCAAAAG TGGGGTGCCACGTTCCCCCACGTACCCAACTACTCTCCCAGTACACAT CCCAATGAGGGACTAGAAGAGAACTACTGTAGGAACCCAGACAATGAT GAACAAGGGCCTTGGTGCTACACTACAGATCCGGACAAGAGATATGAC TACTGCAACATTCCTGAATGTGAAGAGGAATGCATGTACTGCAGTGGA GAAAAGTATGAGGGCAAAATCTCCAAGACCATGTCTGGACTTGACTGC CAGGCCTGGGATTCTCAGAGCCCACATGCTCATGGATACATCCCTGCC AAATTTCCAAGCAAGAACCTGAAGATGAATTATTGCCACAACCCTGAC GGGGAGCCAAGGCCCTGGTGCTTCACAACAGACCCGACCAAACGCTGG GAATACTGTGACATCCCCCGCTGCACAACACCCCCGCCCCCACCCAGC CCAACCTACCAATGTCTGAAAGGAAGAGGTGAAAATTACCGAGGGACC GTGTCTGTCACCGTGTCTGGGAAAACCTGTCAGCGCTGGAGTGAGCAA ACCCCTCATAGGTGA GTCGAC (SEQ ID NO:22),a concatamer of angiostatin having the following amino acid sequence (the intervening sequences are underlined) :
               MVYLSECKTGIGNGYRGTMSRTKSGVACQKWGATFPHVPNYSPSTHPNEGLE ENYCRNPDNDEQGPWCYTTDPDKRYDYCNIPECEEECMYCSGEKYEGKISKTMS GLDCQAWDSQSPHAHGYIPAKFPSKNLKMNYCHNPDGEPRPWCFTTDPTKRWEY CDIPRCTTPPPPPSPTYQCLKGRGENYRGTVSVTVSGKTCQRWSEQTPHRHNRT PENFPCKNLEENYCRNPDGETAPWCYTTDSQLRWEYCEIPSCESSASPDQSDSS VPPEEQTPWQECYQSDGQSYRGTSSTTITGKKCQSEQTPHRGKSMVYLSECKT GIGNGYRGTMSRTKSGVACQKWGATFPHVPNYSPSTHPNEGLEENYCRNPDNDE QGPWCYTTDPDICRYDYCNIPECEEECMYCSGEKYEGKISKTMSGLDCQAWDSQS PHAHGYIPAKFPSKNLKMNYCHNPDGEPRPWCFTTDPTKRWEYCDIPRCTTPPP PPSPTYQCLKGRGENYRGTVSVTVSGKTCQRWSEQTPHRHNRTPENFPCKNLEE NYCRNPDGETAPWCYTTDSQLRWEYCEIPSCESSASPDQSDSSVPPEEQTPWQ ECYQSDGQSYRGTSSTTITGKKCQSEQTPHR(SEQ ID No :23), which is encoded by the following DNA sequence (the *Sal*I sites are underlined, and the intervening sequences are in bold) :
prolactin having the following amino acid sequence: MLPICPGGAARCQVTLRDLFDRAVVLSHYIHNLSSEMFSEFDKRYTHGRGFI TKAINSCHTSSLATPEDKEQAQQMNQKDFLSLIVSILRSWNEPLYHLVTEVR GMQEAPEAILSKAVEIEEQTK (SEQ ID NO:25), which is encoded by the following DNA sequence:
   ATGTTGCCCATCTGTCCCGGCGGGGCTGCCCGATGCCAGGTGACCCTTCGAG ACCTGTTTGACCGCGCCGTCGTCCTGTCCCACTACATCCATAACCTCTCCTC AGAAATGTTCAGCGAATTCGATAAACGGTATACCCATGGCCGGGGGTTCATT ACCAAGGCCATCAACAGCTGCCACACTTCTTCCCTTGCCACCCCCGAAGACA AGGAGCAAGCCCAACAGATGAATCAAAAAGACTTTCTGAGCCTGATAGTCAG CATATTGCGATCCTGGAATGAGCCTCTGTATCATCTGGTCACGGAAGTACGT GGTATGCAAGAAGCCCCGGAGGCTATCCTATCCAAAGCTGTAGAGATTGAGG AGCAAACCTAA (SEQ ID NO:26); and
a concatamer of prolactin having the following amino acid sequence (the intervening sequences are underlined):
   MLPICPGGAARCQVTLRDLFDRAVVLSHYIHNLSSEMFSEFDKRYTHGRGFITK AINSCHTSSLATPEDKEQAQQMNQKDFLSLIVSILRSWNEPLYHLVTEVRGMQE APEAILSKAVEIEEQTKGKSMLPICPGGAARCQVTLRDLFDRAVVLSHYIHNLS SEMFSEFDKRYTHGRGFITKAINSCHTSSLATPEDKEQAQQMNQKDFLSLIVSI LRSWNEPLYHLVTEVRGMQEAPEAILSKAVEIEEQTK (SEQ ID NO:27), which is encoded by the following DNA sequence (the intervening sequences are in bold):

Increase efficacy will occur with concatamers of the anti-angiogenic genes. This will increase the anti-angiogenic dosage level without changing the amount of vector necessary to deliver these genes. Similar to concatamers, a plasmid with two or more promoters, a plasmid with the IRES sequence (internal ribosomal entry site) between two sequences, and an antiangiogenic peptide with a secretory sequence will increase the delivery of genes to the therapeutic target without markedly increasing the DNA concentration. With regards to the concatamers, the concatamers can extend up to approximately 4400 bases in length (the coding region of a large protein), and the number of concatamers possible will depend on the number of bases of a single anti-angiogenic unit.

For fibronectin, the range of concatamers would be about 2 to 66. Although, the maximum number of anti-angiogenic units for the TSPf is about 6, one can increase this concatameric number by deleting the sequences that do not have any anti-angiogenic effects, such as shown below:
ATG(CTGAGGCGGCCTCCCCTATGCTATCACAACGGAGTTCAGTACAGAAATAA CGGTAAAAGATCCCCGTGGTCATCTTGTTCTGTGACATGTGGTGATGGTGTGAT GGTAAAAGAAGTGGTACCCTGTAGACAAGACAGTGGACACCTCCTCCCCAT)ₙT AA (SEQ ID NO:29), where the corresponding amino acid sequence is:
   M(LRRPPLCYHNGVQYRNNEEWTVDSGKSSPWSSCSVTCGDGVITRIGKSSPWD ICSVTCGGGV)ₙ (SEQ ID NO:30), and wherein n in an integer of from 2 to 24. In a similar manner, the concatameric number of the platelet factor-4 peptide, somatostatin inhibitor, angiostatin, and prolactin can be increased.

Since more than one anti-angiogenic pathway exists, concatamers consisting of two or more types of inhibitor are believed to be more effective than the homogenous concatamers. For example, heterogeneous concatamers of TSPI and the fibronectin inhibitors can be inserted into the same vector. An example of a heterogenous concatamer useful is present invention is as follows:
ATG(CTGAGGCGGCCTCCCCTATGCTATCACAACGGAGTTCAGTACAGAAATAA CGGTAAAAGATCCCCGTGGTCATCTTGTTCTGTGACATGTGGTGATGGTGTGAT GGTAAAAGAAGTGGTACCCTGTAGACAAGACAGTGGACACCTCCTCCCCAT)ₓ( TATATTGGTTCTCGTGGTAAAAGA)yTAA (SEQ ID NO:31). The first parenthetical represents the nucleotide sequence of TSPf, whereas the second parenthetical represents the anti-angiogenic fragment isolated from fibronectin, wherein x and y represent the number of repeats of TSPI and fibronectin, respectively. Again, the number of bases delineated by the summation of x + y will generally not exceed 4400 bases.

The above heterogeneous concatamers need not be limited to only anti-angiogenic peptides. For example, the protein angiostatin or the large polypeptide fragment of prolactin can be modified with with the above-mentioned genes which encode anti-angiogenic pepetides. Again, the concatameric number will vary depending on the number of nucleotide bases of the unit angiogenic inhibitor. In this concatomer of large and small anti-angiogenic inhibitors, the ratio of of large to small inhibitors is 0.1 to 0.9, preferably 1:1.

A translational start signal Met, has been included in all of the above peptides; and a transcriptional stop codon (TAA) has been included in all of the above DNA sequences.

The *Sal*I sites indicated in the above-sequences are a useful cloning tool for insertion of the DNA into BAP vector, which is known to useful in expressing proteins efficiently *in vivo* from the β-actin promoter (Ray et al, *Genes Dev.,* 5:2265-2273 (1991)). Other restriction sites can be incorporated into the DNA for cloning into other vectors.

Other useful vectors for gene therapy which can be employed in the present invention include plasmids with a simian viral promoter, e.g., pZeoSV (Invitrogen); or the CMV promoter, e.g., pcDNA3, pRc/CMV or pcDNA1 (Invitrogen). Plasmids with a CMV promoter may contain an intron 5' of the multiple cloning site (Zhu et al, *supra*). Plasmids containing the BGH terminator instead of the viral SV40 polyA terminator, e.g., pcDNA3, pRc/CMV, pRc/RSV (Norman et al, IBC's 5th Annual Meeting (1995); and Invitrogen vectors), can also be employed in the present invention so as to increase the expression of the tumor suppressor gene and the anti-angiogenic peptide in cells. As stated previously, a vector containing two or more promoters will greatly enhance the therapeutic efficacy. Vectors containing the IRES sequence which allows the translation of two different coding genes to occur from one mRNA transcript will also significantly increase the efficacy of the therapy.

Expression of the DNA encoding the tumor suppressor protein and the DNA encoding the anti-angiogenic peptide can be achieved using a variety of promoters, and the particular promoter employed is not critical to the present invention. For example, the promoter can be a generalized promoter, such as the β-actin promoter, a simian viral promoter, or the CMV promoter, or a tissue specific promoter, such as the α-fetal protein promoter which is specific for liver (Kaneko et al, *Cancer Res*., 55:5283-5287 (1995), the tyrosinase promoter which is specific for melanoma cells (Hughes et al, *Cancer Res.*, 55:3339-3345 (1995); or the enolase promoter which is specific for neurons (Andersen et al, *Cell. Molec. Neurobiol.,* 13:503-515 (1993)).

The particular amount of DNA included in the cationic liposomes of the present invention is not critical. Generally, the amount of total DNA is in the range of about 0.005 to 0.32 µg/pM of liposome, preferably 0.045 to 0.08 µg/pM of liposome.

The DNA encoding a tumor suppressor gene is generally present in an amount of from 0.0025 to 0.16 µg/pM of liposome, preferably 0.028 to 0.04 µg/pM of liposome. The DNA encoding an anti-angiogenic peptide is generally present in an amount of from 0.0025 to 0.16 µg/pM of liposome, preferably 0.028 to 0.04 µg/pM of liposome.

The mole ratio of the DNA encoding the tumor suppressor gene to the DNA encoding the anti-angiogenic peptide is not critical to the present invention. Generally, the mole ratio is between 1:5 to 5:1, preferably about 1 to 1.

The DNA encoding the tumor suppressor gene and the anti-angiogenic peptide may be contained on the same vector, or on separate vectors.

Cationic liposomes are prepared similarly to other liposomes. In brief, the cationic lipid with/or without DOPE are dissolved in a solvent, e.g., chloroform. The lipids are then dried in a round bottom flask overnight on a rotary evaporator. The resulting lipids are then hydrated with sterile water over a 1 hr period so form large mutilamellar vesicle liposomes. To decrease the size of the liposomes, one may sonicate or pass the liposomes back and forth through a polycarbonate membrane. The DNA is then added to a solution containing the liposomes after their formation.

In another embodiment, the above-described objects of the present invention have been met by a method for inhibiting tumor growth in a subject comprising administering to a tumor-bearing subject a cationic liposome:DNA complex comprising DNA encoding a tumor suppressor gene and DNA encoding an anti-angiogenic peptide.

In a preferred embodiment the cationic liposome:DNA complex additionally comprises DNA encoding a tumor suppressor protein.

In a further preferred embodiment the cationic polymer :DNA complex additionally comprises DNA encoding a tumor suppressor protein. In a further preferred embodiment the cationic liposomes in the cationic liposome:DNA complex are comprised of one cationic lipid (i.e.- 1,2-dioleolyl-sn-glycero-3-ethyl-phosphocholine, 1,2-dimyristoyl-sn-glycero-3-ethylphosphocholine, and 2,3-diol-eyloxy)propyl-N,N,N- tri-methyl-ammonium chloride) and may also be comprised of polyethylimine glycol (i.e., a pegylated lipid-1,2-diacy-sn-glycero-3-phospho-ethanolamine-N-Poly(ethylene glycol) 2000) and a targeted ligand (the amino acid peptide RGD, ferritin, or antibodies targeted toward to HER2).

In a further preferred embodiment of the inventionsaid cationic liposomes in said complexes are comprised of one cationic polymer polyethylimine, polylysine, polyhisitidine, and Superfect) and may also be comprised of polyethylene glycol and a targeted 1 ligand (the amino acid peptide RGD, ferritin, or antibodies targeted toward to HER2, CEA).

In a further preferred embodiment said tumor suppressor protein in said complexes is p53.

In a further preferred embodiment of the invention said anti-angiogenic peptide in the said complexes is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

In a further preferred embodiment the said DNA encoding an anti-angiogenic peptide used in the said cationic liposome:DNA complexe is present in an amount of from 0.0025 to 0.16 µg/pM of liposome.

In a further preferred embodiment the said DNA encoding an anti-angiogenic peptide in the said cationic polymer:DNA complex is present in an amount of from 0.016 to 0.33 µg/µg of polymer.

In a further preferred embodiment the said DNA encoding a tumor suppressor protein in the said complexes is present in an amount of from 0.0025 to 0.16 µg/pM.

In a further preferred embodiment of the present invention said DNA encoding a tumor suppressor protein in the said complexes is present in an amount of from 0.016 to 0.33 µg/pM.

A further embodiment of the present invention is the provision of the use of a cationic polymer:DNA complex comprising DNA encoding an anti-angiogenic peptide for the production of a medicament for inhibiting tumor growth in a subject which preferably comprises administering the same to a tumor-bearing subject.

In a further preferred embodiment of the present invention the said complex in the said use additionally comprises DNA encoding a tumor suppressor protein.

In a further preferred embodiment of the present invention the said cationic liposome in the said use is (i.e.-1,2-dioleolyl-sn-glycero-3-ethylphosphocholine, 1,2-dimyristoyl-sn-glycero-3-ethylphosphocholine, and 2,3-diol-eyloxy)propyl- N,N,N-trimethylammonium chloride and may also be comprised of polyethylene glycol (i.e., a pegylated lipid-1,2 -diacy-sn-glycero-3-phosphoethanolamine-N-[Poly-(ethylene glycol) 2000) and a targeted ligand (the amino acid peptide RGD, ferritin, or antibodies targeted toward to HER2).

In a further preferred embodiment of the present invention the said cationic polymer in the said use is (*i.e.* (polyethylimine Polycat57, polylysine, polyhisitidine, and Superfect) and may also be comprised of polyethylene glycol and a targeted ligand (the amino acid peptide RGD, ferritin, or antibodies targeted toward to HER2).

In a further preferred embodiment of the present invention the said tumor suppressor protein used in the said cationic polymer is p53.

In a further preferred embodiment the said anti-angiogenic peptide used for the said cationic polymer is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

In a further preferred embodiment of the present invention the said DNA encoding an anti-angiogenic peptide used in the cationic complex is present in an amount from 0.0025 to 0.16 µg/pM of liposome or 0.016 to 0.33 µg/µg of polymer.

In a further preferred embodiment of the present invention the said DNA encoding a tumor suppressor protein used in the said complex is present in an amount of from 0.0025 to 0.16 µg/pM of liposome or 0.016 to 0.33 µg/µg of polymer.

In a further embodiment of the present invention the said cationic polymer:DNA complex comprising a plasmid with one or more promoters expressing either the same gene product (SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30) or combinations of these gene products.

In a further embodiment of the present invention the cationic liposome:DNA complex comprising a plasmid with an intervening internal ribosomal entry site sequence between two genes (SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, and SEQ ID NO:29) that are either the same or different.

In a further preferred embodiment the said anti-angiogenic protein in the said cationic liposome:DNA complex or the said cationic polymer:DANN complex is secretory from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

The particular type of tumor which can be treated in the present invention is not critical thereto. Examples of tumors which can be treated in accordance with the present invention include solid tumors, e.g., lung, colon, brain, breast and melanoma tumors. All of these tumors are very dependent on blood supply to sustain their growth.

The particular mode of administering the cationic liposome:DNA complex of the present invention is not critical thereto. Examples of such modes include intravenous, subcutaneous and intratumoral injections.

Intravenous injection is the preferred administration mode since there is better distribution to the developing blood vessels of the tumor.

The amount of cationic liposome:DNA complex to be administered will vary depending upon the age, weight, sex of the subject, as well as the tumor volume and rate of tumor growth in the subject. Generally, the amount to be administered will be about 5 to 60 µg, preferably about 9 to 16 µg.

The following examples are provided for illustrative purposes only, and are in no way intended to limit the scope of the present invention.

### EXAMPLE 1

### Production of DNA Vectors

### A. TSPI Vector

The coding region of the TSPI gene is well-known (GB Accession code-X14787). The TSPI gene was inserted into the XbaI site of BAP vector (Ray et al, *supra*), so as to give rise to TSPI vector, wherein expression of the TSPI gene is controlled by the β-actin promoter.

More specifically, TSPI cDNA and Bluescript plasmid (Promega) were digested with *Hind*I and *Xba*I, and then the TSPI cDNA was ligated into Bluescript. Next, Bluescript containing the TSP cDNA and BAP vector were digested with *Sal*I and *Bam*HI, and TSPI cDNA inserted in the *Xba*I site of BAP vector. The correct orientation of the TSPI gene in BAP vector was confirmed by DNA sequencing.

### B. TSPf Vector

TSPf vector is a vector containing a DNA fragment of the TSPI gene which has the two anti-angiogenic domains (nucleotides 992-1650) (Tolsma et al, *supra*), and a start codon and a stop codon.

The DNA fragment was prepared by PCR using thrombospondin I cDNA as template, and 100 pmoles of each of the following primers 5'-TAGGTCTAGAATGACTGAAGAGAACAAAGAG-3' (SEQ ID NO:24); and 5'-ATGGTCTAGA**TTA**GAGACGACTACGTTTCTG-3' (SEQ ID NO:25) so as to amplify nucleotides 1013 to 1650 of the TSPI gene. Both primers contain *Xba*I sites (underlined), the first primer contains an ATG start codon (in bold), and the second primer contains a TTA stop codon (in bold).

The resulting 638 base pair fragment of the TSPI gene (hereinafter "TSPf") encodes the peptides that are known to be angiogenic inhibitors (Tolsma et al, *supra*).

After amplification, the DNA fragment was purified, digested with *Xba*I, and the digested fragment inserted into the *Xba*I site of BAP vector such that the expression of the TSPf gene was controlled by the β-actin promoter (Ray et al, *supra;* and Lesoon-Wood et al, *Human* Gene *Ther*., 6:395-405 (1995)). The correct orientation of the fragment in BAP vector was verified by digestion with *Bam*HI, and confirmed by DNA sequencing.

### C. p53 Vector

The coding sequence of the p53 gene was cut from plasmid plSVhp53c62 (Zakut-Houri et al, *EMBO J.*, 4:1251-1255 (1985)) with *Xba*I, and inserted into the multiple cloning sites of pGEM3Z vector (Promega, Madison, WI). Digestion of the resulting vector with *Sal*I and *Bam*HI generated a 1900 bp fragment that was then inserted into the *Sal*I and *Bam*HI sites of BAP vector such that expression of the p53 gene was controlled by the β-actin promoter. The correct orientation of the p53 gene in BAP vector was confirmed by DNA sequencing.

### EXAMPLE 2

### Preparation of Cationic Liposome:DNA Complexes

A DOTMA:DOPE liposome mixture is known to efficiently transfect endothelial cells *in vitro* (Tilkins et al, *Focus,* 16:117-119 (1994)). Accordingly, liposome:DNA complexes were prepared using DOTMA:DOPE, in a 1:1 ratio, essentially as described by Debs et al, *J. Biol. Chem.,* 265:10189-10192 (1990). Similar liposomes preparations can be prepared by mixing, at a 1:1 ratio, DOPE with other cationic lipids, such as, 1,2-dioleolyl-sn-glycero-3-ethylphophocholine, and 1,2-dimyristoyl-sn-glycero-3-ethylphophocholine.

More specifically, a mixture of 400 nmoles of the DOTMA and DOPE were dried overnight on a rotary evaporator. Then, the lipids were rehydrated with 1.5 ml of water for 2 hrs. Next, the milky liposome preparation was sonicated with a bath sonicator until clear. The resulting liposome preparation was then passed through a 50 nm polycarbonate filter between 15 to 20 times with a LipsoFast-Basic extruder (Avestin, Ottawa, On).

The DNA employed was either (1) empty BAP vector; (2) TSPI vector alone; (3) TSPf vector alone; (4) p53 vector alone; (5) p53 vector + TSPI vector; or (6) p53 vector + TSPf vector.

The DNA was prepared with the maxi Qiagen kits (Qiagen Inc., Chatsworth, Ca), and washed twice in 70% (v/v) ethanol. The DNA was then dialyzed against water for 24 hrs to removed any remaining salt.

About 400 pmols of the liposome preparation was gently mixed with between 18 to 35 µg of total DNA in an Eppendorf tube. This amount in each eppendorf tube was sufficient for two injections. The same amount of DNA was injected in the combination therapies as in the single treatment regimens. For example, if 16 µg of DNA in the combination therapy (8.0 µg of p53 + 8.0 µg of TSPf) was injected into each mouse of one group, then 16 µg of p53 was injected into each mouse of a second group.

### EXAMPLE 3

### Anti-Angiogenic Effect of Cationic Liposome:DNA Complexes

The anti-angiogenic effects of the cationic liposome:DNA complexes obtained in Example 2 were evaluated in mice containing MDA-MB-435 breast cancer tumors (American Type Tissue Culture, Bethesda, MD), which are p53 deficient.

More specifically, after administering the anesthetic, Metofane, to 126 female athymic nude mice (NCI), the mice were injected with 2.0 x 10⁵ MDA-MB-435 tumor cells into the mammary fat pad using a stepper (Tridak) and a 27.5 g needle. Two weeks later, the mice whose tumors grew were divided into various treatment regimens, 18 mice per each regimen. The treatment regimens were as follows: (1) untreated; (2) empty BAP vector; (3) TSPI vector alone; (4) TSPf vector alone; (5) p53 vector alone; (6) p53 vector + TSPI vector; and (7) p53 vector + TSPf vector. The mice received two intravenous injections, the first injection 14 days after the malignant cells had been implanted into the mice, and the second injection 24 days after the malignant cells had been implanted into the mice. The first injection consisted of 200 pmoles of the liposomes complexed with 16 µg of total DNA. The second injection consisted of 200 pmols of the liposomes complexed with 8.0 µg of total DNA. The sizes of the tumors were measured 7 days after the second injection. The results are shown in Table 1 below.

**TABLE 1**

| Anti-tumor Effects of TSPI and TSPf | |
|---|---|
| Putative Anti-tumor Genes | Tumor Size (mm³) |
| Untreated | 113.5±6.41 |
| BAP | 102.9±6.83 |
| TSPI | 103.2±8.96 |
| TSPf | 89.4±11.06 |
| p53 | 80.1±12.7* |
| p53 + TSPI | 82.9±6.95* |
| p53 + TSPf | 53.2±8.37** |

| | |
|---|---|
| * p53 or p53 + TSPI vs. untreated, p<0.05 ** p53 + TSPf vs. untreated or BAP, p<0.01 | |

As shown in Table 1 above, the p53-treated group was found to be statistically different from the untreated group (p<0.05) after 2 injections. However, the p53 treated group was not statistically different from the empty BAP vector group. This was similar to the results described by Lesoon-Wood et al, *Human Gene Ther*., 6:395-406 (1995), in which p53 was not statistically different from the empty BAP vector group until after 5 injections.

However, p53 in combination with TSPf reduced tumors more effectively than p53 alone. That is, after just 2 injections of this combination therapy, there was a 35% further reduction in tumor growth compared to p53 alone. The combination group was statistically different from both the untreated and the empty BAP vector groups (p<0.01). Although TSPf by itself was slightly less effective than p53, TSPf was, unexpectedly, substantially more effective than TSPI. In fact, the full length TSPI-treatment group had no more effect than either the empty vector or the untreated groups. This was unexpected for several reasons: 1) both the full length and the fragment of thrombospondin I contained the anti-angiogenic peptide 2) in a previous ex vivo study (Weinstat-Saslow et al, supra), full length thrombospondin I was effective in inhibiting tumor growth, and 3) full length thrombospondin I has a secretory sequence presumably so that the secreted protein can inhibit endothelial proliferation, whereas the thrombospondin I fragment does not contain a secretory sequence.

Regardless of whether there is a secretory sequence, one would predict prior to the present invention that the liposome:antiangiogenic gene would not be an effective antitumor therapy. As taught by Lesoon-Wood et al., the transfection efficiency of the tumor with cationic liposomes was very low. In fact, it could not be quantitated with a primer extension method. We know from the teaching of Weinstat-Saslow et al. that high levels of expressed TSPI in 100% of the tumor cells reduces the tumor growth by only 60% in an ex vivo system. Extrapolating from these findings, a relatively high transfection efficiency of 20% with the liposome: antiangiogenic genes would have resulted in a marginal reduction (20%/100% X 60% reduction = 12%) of the tumor. This amount of tumor reduction would not have resulted in statistical differences with the liposome:antiangio-genic gene complexes. A transfection efficiency of the tumor above 10% would have easily been measurable with a variety of techniques including the primer extension method (used by Lesoon-Wood et al.). It has been determined that the transfection efficiency of the tumor is less than 5% with these cationic liposomes.

Hence, it was clearly unobvious that DNA encoding an anti-angiogenic peptide alone would be an effective anti-tumor agent *in vivo,* based upon teachings that an anti-angiogenic peptide is an effective anti-tumor agent (Tolsma et al and Bouck et al), and based upon the teachings that DNA encoding a full-length anti-angiogenic protein is an effective anti-tumor agent *ex vivo* (Weinstat-Saslow et al).

A second experiment was carried out to determine whether the combination therapy of p53 and TSPf was effective at lower dosages, and to confirm that the combination of p53 and TSPf reduced the tumor size considerably more than p53 alone.

More specifically, 36 mice were injected with 2.0 x 10⁵ MDA-MB-435 tumor cells into the mammary fat pad. Two weeks later, the mice whose tumors grew were divided into various treatment regimens, 18 mice per each regimen. The treatment regimens were as follows: (1) empty BAP vector; (2) p53 vector alone, and (3) p53 vector + TSPf vector. The mice were injected intravenously with 200 pmols of the liposomes complexed with 8.0 µg of total DNA. Subsequently, the mice were treated in the same manner with 200 pmols of the liposomes complexed with 12 µg of total DNA for the next 4 injections. Ten days elapsed between each injection. The sizes of the tumors were measured before each injection and 7 days after the last injection. The results are shown in Table 2 below:

**TABLE 2**

| Anti-tumor Effects of p53 and TSPf | |
|---|---|
| Putative Anti-tumor Genes | Tumor Size (mm³) |
| BAP | 855±345 |
| p53 | 616±142 |
| p53 + TSPf . | 265±133* |

| | |
|---|---|
| ⁺ p53 + TSPf vs. BAP, p<0.02 | |

As shown in Table 2 above, the combination therapy with p53 and TSPf was statistically different from BAP, whereas the p53 alone treatment was not. This experiment confirmed that p53 and TSPf were more effective than p53 alone. Furthermore, a different dosage regimen, without an initial booster dose of 16 µg of DNA as used in the experiment in Table 1, accentuated the difference between the combination treatment and the p53 alone treatments.

Next, MCF7 cells (American Type Tissue Culture, Bethesda, MD), which are a breast cancer cell line with two normal p53 alleles, were evaluated as described above except that 4.0 x 10⁶ cells were injected into the mice; and the third injection contained 12 µg of the DNA. Each injection was 10 days apart. Nine mice were injected with each of the following treatments except for regimen (1), in which 8 mice were treated: (1) untreated; (2) BAP; (3) p53; and (4) p53 + TSPf. The sizes of the tumors were measured 7 days after the third injection. The results are shown in Table 5 below.

**TABLE 3**

| Effect of p53 and TSPf on MCF7s Cells | |
|---|---|
| Putative Anti-tumor Genes | Tumor Size (mm³) |
| Untreated | 124.6±7.3 |
| BAP | 136±16.8 |
| p53 | 83.1±13.6* |
| p53 + TSPf | 69.0±13** |

| | |
|---|---|
| * p53 vs. untreated or BAP, p<0.05 ** p53 + TSPf vs. untreated or BAP, p<0.01 | |

As shown in Table 3 above, the most effective therapy against MCF7s was p53 and TSPI. The significance level for the p53 + TSPf therapy was greater than for p53 alone when they were compared against either the untreated or the BAP groups.

The above experiment verifies that p53 and TSPfI decreased the MCF7s tumor more than the p53 treated or the untreated groups. 4 X 10⁵ MCF7 cells were injected bilaterally into the mammary fat pads of the 28 nude mice. After two weeks of growth, these mice were randomly divided into four groups: 1)empty vector, 2) p53, 3) p53 + TSPf , and 4) untreated. The mice received one injection of 200 pmoles of liposomes complexed with 14 ugs of DNA, and the tumors from the various treatment groups were measured 10 days after the treatment. The results are shown in Table 4 below.

**Table 4**

| Putative Anti-tumor Genes | Tumor Size (mm³) |
|---|---|
| Empty vector- | 54.7±4.0 |
| p53 | 45.5±5.0 |
| p53 + TSPf | 33.9±3.6* |
| Untreated | 61.9+8.3 |

| | |
|---|---|
| *, p53 + TSPf vs Untreated, p<.025 | |

As shown in Table 4, the additional reduction of the tumor by the combined use of p53 and TSPf (also in Tables 1, 2, and 4 above) compared to the use of p53 only, suggest that TSPf and p53 have different mechanisms of action. Although this does not preclude that the target of p53 is the vasculature of the tumor, the mechanism of inhibition of the tumor by p53 is not known at present. However, any mechansim of tumor inhibition by p53 and/or thrombospondin I must account for the low transfection efficiency of the tumor. Again, with a liposome complexed to a chloramphenicol acetyltransferase marker, it has been demonstrated that less than 5% of the tumor derived from MDA-MB-435 cells was transfected with the marker gene.

Besides p53 and the antiangiogenic fragment of thrombospondin I, we determined that liposomes complexed to DNA encoding the laminin peptide inhibits tumor growth. More specifically, after administering the anesthetic, Metofane, to 24 female athymic nude mice, the mice were injected with 3.0 x 10⁵ MDA-MB-435 tumor cells into the mammary fat pad using a stepper and a 27.5 g needle. Two weeks later, the mice whose tumors grew were divided into various treatment regimens, 8 mice per each regimen. The treatment regimens were as follows: (1) BAP, (2) laminin, and (3) p53 + laminin. The mice were injected intravenously with 200 pmols of the liposomes complexed with 12.5 mgs of total DNA 6.25 mg of each vector when a combination was used. The mice then received 3 injections, each 10 days apart. The tumors were measured at the time of each injection and at the time of the last injection. The results are shown in Table 7 below.

**TABLE 5**

| Putative Anti-tumor Genes | Tumor Size (mm³) |
|---|---|
| BAP | 345 ± 23.5 |
| Laminin peptide | 280 ± 32.4 |
| Laminin peptide + p53 | 192 ± 10.5* |

| | |
|---|---|
| * BAP vs. Laminin peptide + p53, p<0.05 | |

As shown in Table 5 above, cationinc liposomes containing a combination of DNAs encoding laminin peptide + p53 was unexpectedly more effective in reducing tumor growth than when DNA encoding the anti-angiogenic peptide was used alone. Thus, the addition of a tumor suppressor gene, p53, enhances the anti-tumor effect of the anti-angiogenic peptide.

In vitro assays indicate that cationic polymers will significantly improve the present therapy. When a carrier such as a cationic lipid was used in this in vitro assay, the inhibitory effect (of the genes p53, TSPf, and the combination of p53 and TSPf) was marginal whereas another vector, Superfect (a cationic polymer), was much more effective as a carrier. This is because Superfect was 15 times more effective than the cationic liposomes in transfecting endothelial cells with the CAT marker. The cationic liposomes used in this section was DOSPER (Boerhinger), which of the 14 lipids tested gave the best results. Included in this panel of 14 lipids that we tested was lipofectin (BRL) which is a mixture of DOTMA/DOPE that we have used in an in vivo study. In brief, we plated 1X10⁶ Huvec cells into each well of a 6 well plate. 25uls of Superfect complexed with 2 ugs of DNA was added to each plate 24 hours after the initial seeding of the cells. 36 hours after the transfection, the cells were lysed and the amount of CAT protein was assayed.

**Table 6**

| Vectors | Activity(DPMs/protein) |
|---|---|
| Cationic liposomes with BAP | 31.1±7.2 |
| Cationic liposomes with CAT | 682±129 |
| Superfect with BAP | 21.4±0.458 |
| Superfect with CAT | 10816±687* |

| | |
|---|---|
| p<0.001, Superfect-CAT vs Cationic liposome-CAT | |

This experiment clearly demonstrates that this cationic polymer is a superior in the transfection of endothelial cells, which is a likely target of the therapeutic gene. We have found that Superfect is a better transfection agent than cationic liposomes for many different cell lines. Since Superfect which is a cationic polymers is such an efficient carrier of DNA, this underscores possibility that non-viral carriers as a class of carriers will be effective in decreasing the tumor size. As a result, other non-viral carriers besides liposomes should be included in this patent.

Transfection of Huvec Cells with various inhibitors was as follows. 1.0 x 10⁵ Huvec cells (Clonetics), a human endothelial cell line, were plated into each well of a 6-well plate, and placed in a CO₂ incubator at 37°C. Twenty-four hours later, the cells were transfected with 25 ml of Superfect (Qiagen), a cationic polymer, complexed to 2.0 mg of various DNA vectors, i.e., (1) BAP vector; (2) p53 vector; (3) TSPf vector; and (4) p53 vector TSPf vector. After the cells were exposed for 2 hours to this complex at 37°C, the media was removed, and replaced with fresh EGM media (Clonetics, Inc.). containing 10% (v/v) fetal calf serum, and 1.0% (w/v) glutamine, and the cells placed in a CO₂ incubator at 37°C. Twenty-four hours later, the cell number in each plate was determined by the 3-(4,5-dimethylthiazol-2-yl)--(3-carboxymethoxyphenyl)- 2-,5-di- phenyltetetra-zolium bromide (MTS) assay described by Butke et al, *J. Immunol. Methods,* 157:233-240 (1993).

Figure shows the effect of different treatment groups on endothelial cells in vitro. it was found that the percentage of BAP control is significantly decreased when using BAP-p53 and BAPf-TSPf as tretment groups. A further synergistic decrease is achieved when using BAP-p53/TSPf a the treatment group.

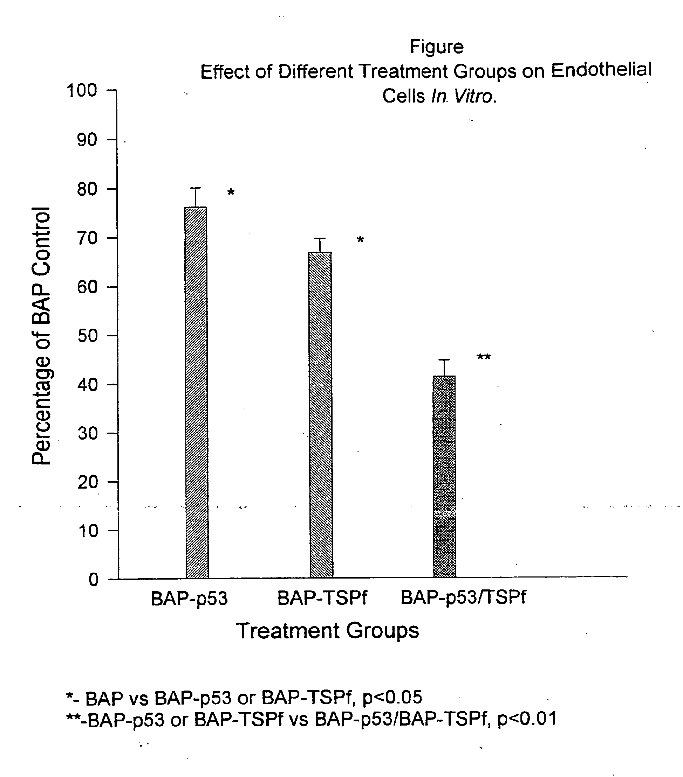

## Claims

1. A cationic liposome:DNA complex comprising DNA encoding an anti-angiogenic peptide said complex additionally comprises DNA encoding a tumor suppressor protein wherein said tumor suppressor protein is p53 and wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

2. The complex of Claim 1, wherein said cationic liposomes are comprised of one cationic lipid, preferably 1,2-dioleoyl-sn-glycero-3-ethylphosphocholine,1,2-dimyristoyl-sn-glycero-3-ethylpho s-phocholine, and N-[1(2,3-dioleoyloxy)propyl]-N,N,N- trimethyl-ammonium chloride and may also be comprised of polyethylene glycol, preferably a pegylated lipid-1,2-diacyl-sn-glycero-3-phospho-ethanolamine-N-Poly(ethylene glycol) 2000) and a targeted ligand , preferably the amino acid peptide RGD, ferritin, or antibodies targeted toward to HER2.

3. The complex of Claim 1, wherein said cationic liposomes are comprised on its outer membrane through bonding with one cationic polymer poly-ethylenimine, preferably Polycat57, polylysine, polyhistidine, and Superfect and may also be comprised of polyethylene glycol and a targeted ligand, preferably the amino acid peptide RGD, ferritin, or antibodies targeted toward to HER2, CEA.

4. The complex of claim 1, wherein said DNA encoding a tumor suppressor protein is present in an amount of from 0.0025 to 0.16 µg/pM of liposome.

5. The complex of Claim 1, wherein said DNA encoding a tumor suppressor protein is present in an amount of from 0.016 to 0.33 µg/pM of liposome.

6. The use of a complex for the production of a medicament to inhibit tumor angiogenesis according to claim 1, wherein said cationic liposome includes preferably 1,2-dioleoyl-sn-glycero-3- ethylphosphocholine, 1,2-dimyristoyl-sn-glycero-3- ethylphosphocholine, and N[1-(2,3-dioleoyloxy)propyl]- N,N,N- trimethylammonium chloride and may also be comprised of polyethylene glycol preferably a pegylated lipid-1,2 -diacyl-sn-glycero-3-phosphoethanolamine-N-[Poly-(ethylene glycol) 2000 and a targeted ligand preferably the amino acid peptide RGD, ferritin, or antibodies targeted toward to HER2.

7. A cationic polymer:DNA complex comprising the DNA encoding an antiangiogenic peptide, wherein said complex additionally comprises DNA encoding a tumor suppressor protein wherein said tumor suppressor protein is p53 wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

8. The complex of Claims 7, wherein said DNA encoding an anti-angiogenic peptide is present in an amount of from 0.016 to 0.33 µg/µg of polymer.

9. The complex of Claim 7, wherein said DNA encoding a tumor suppressor protein is present in an amount of from 0.0025 to 0.16 µg/pM.

10. The complex of Claim 7, wherein said DNA encoding a tumor suppressor protein is present in an amount of from 0.016 to 0.33 µg/pM.

11. Use of a cationic polymer:DNA complex of claim 7 comprising DNA encoding an anti-angiogenic peptide for the production of a medicament for inhibiting tumor growth in a subject which preferably comprises administering the same to a tumor-bearing subject.

12. The use of Claim 11, where said complex additionally comprises DNA encoding a tumor suppressor protein.

13. The complex of claim 1, wherein said cationic polymer is selected from the group consisting of polyethylenimine preferably Polycat 57, polylysine, polyhistidine, and Superfect and may also be comprised of polyethylene glycol and a targeted ligand preferably the amino acid peptide RGD, ferritin, or antibodies targeted toward to HER2.

14. The use of Claim 11, wherein said anti-angiogenic peptide is selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, and SEQ ID NO:30.

15. The use of Claim 11 or Claim 12 wherein said DNA encoding an anti-angiogenic peptide is present in an amount from 0.016 to 0.33 µg/µg of polymer.

16. The use of Claims 11 or 12, wherein said DNA encoding a tumor suppressor protein is present in an amount of from 0.016 to 0.33 µg/µg of polymer.

## Patentansprüche

1. Kationischer Liposom-DNS-Komplex, umfassend DNS, welche für ein antiangiogenes Peptid kodiert, wobei der Komplex zusätzlich DNS umfasst, welche für ein Tumorsuppressorprotein kodiert, **dadurch gekennzeichnet, dass** das Tumorsuppressorprotein p53 ist, wobei das antiangiogene Peptid ausgewählt ist aus der Gruppe bestehend aus: SEQ-ID Nr.: 1, SEQ-ID Nr.: 3, SEQ-ID Nr.: 5, SEQ-ID Nr.: 7, SEQ-ID Nr.: 9, SEQ-ID Nr.: 11, SEQ-ID Nr.: 13, SEQ-ID Nr.: 15, SEQ-ID Nr.: 17, SEQ-ID Nr.: 19, SEQ-ID Nr.: 21, SEQ-ID Nr.: 23, SEQ-ID Nr.: 25, SEQ-ID Nr.: 27 und SEQ-ID Nr.: 30.

2. Komplex nach Anspruch 1, **dadurch gekennzeichnet, dass** die kationischen Liposome aus einem kationischen Lipid bestehen, vorzugsweise 1,2-Dioleoyl-sn-glycero-3-ethylphosphocholin, 1,2-Dimyristoyl-sn-glycero-3-ethylphosphocholin und N- [1- (2,3-Dioleyloxy)-propyl]-N,N,N-trimethylammoniumchlorid und ebenso aus Polyethylenglykol bestehen können, vorzugsweise ein pegyliertes Lipid - 1,2-Diacyl-sn-glycero-3-phosphoethanolamin-N-(Polyethylenglykol-2000) und einen gerichteten Liganden, vorzugsweise das Aminosäurepeptid RGD, Ferritin oder Antikörper, gerichtet gegen HER2.

3. Komplex nach Anspruch 1, **dadurch gekennzeichnet, dass** die kationischen Liposome an ihrer äußeren Membran die Bindung an ein kationisches Polymer, Polyethylenimin, umfassen, vorzugsweise Polycat57, Polylysin, Polyhistidin und Superfect und auch Polyethylenglykol und einen gerichteten Liganden, vorzugsweise das Aminosäurepeptid RGD, Ferritin oder Antikörper, gerichtet gegen HER2 und CEA, umfassen kann.

4. Komplex nach Anspruch 1, **dadurch gekennzeichnet, dass** die DNS, die für ein Tumorsuppressorprotein kodiert, in einer Menge von 0,0025 bis 0,16 µg/pM vorliegt.

5. Komplex nach Anspruch 1, **dadurch gekennzeichnet, dass** die DNS, die für ein Tumorsuppressorprotein kodiert, in einer Menge von 0,016 bis 0,33 µg/pM vorliegt.

6. Verwendung eines Komplexes zur Herstellung eines Medikaments um die Tumorangiogenese gemäß Anspruch 1 zu inhibieren, **dadurch gekennzeichnet, dass** das kationische Liposom vorzugsweise 1,2-Dioleoyl-sn-glycero-3-ethylphosphocholin, 1,2-Dimyristoyl-*sn*-glycero-3-ethylphosphocholin und N-[1-(2,3-Dioleoyloxy)-propyl]-N,N,N-trimethylammoniumchlorid einschließt und auch Polyethylenglykol umfassen kann, vorzugsweise ein pegyliertes Lipid - 1,2-Diacyl-*sn*-glycero-3-phosphoethanolamin-N-(Polyethylenglykol-2000) und einen gerichteten Liganden, vorzugsweise das Aminosäurepeptid RGD, Ferritin oder Antikörper, gerichtet gegen HER2.

7. Kationischer Polymer-DNS-Komplex, umfassend die DNS, welche für ein antiangiogenes Peptid kodiert, **dadurch gekennzeichnet, dass** der Komplex zusätzlich DNS umfasst, die für ein Tumorsuppressorprotein kodiert, wobei das Tumorsuppressorprotein p53 ist und wobei das antiangiogene Peptid ausgewählt ist aus der Gruppe bestehend aus: SEQ ID Nr.: 1, SEQ ID Nr.: 3, SEQ ID Nr.: 5, SEQ ID Nr.: 7, SEQ ID Nr.: 9, SEQ ID Nr.: 11, SEQ ID Nr.: 13, SEQ ID Nr.: 15, SEQ ID Nr.: 17, SEQ ID Nr.: 19, SEQ ID Nr.: 21, SEQ ID Nr.: 23, SEQ ID Nr.: 25, SEQ ID Nr.: 27 und SEQ ID Nr.: 30.

8. Komplex nach Anspruch 7, **dadurch gekennzeichnet, dass** die DNS, die für antiangiogenes Peptid kodiert, in einer Menge von 0,016 bis 0,33 µg/µg Polymer vorliegt.

9. Komplex nach Anspruch 7, **dadurch gekennzeichnet, dass** die DNS, die für ein Tumorsuppressorprotein kodiert, in einer Menge von 0,0025 bis 0,16 µg/pM vorliegt.

10. Komplex nach Anspruch 7, **dadurch gekennzeichnet, dass** die DNS, die für ein Tumorsuppressorprotein kodiert, in einer Menge von 0,016 bis 0,33 µg/pM vorliegt.

11. Verwendung eines kationischen Polymer-DNS-Komplexes nach Anspruch 7, umfassend DNS, welche für ein antiangiogenes Peptid kodiert, zur Herstellung eines Medikaments zur Inhibierung des Tumorwachstums bei einem Individuum, welche vorzugsweise die Verabreichung desselben an ein von einem Tumor befallenes Individuum umfasst.

12. Verwendung nach Anspruch 11, **dadurch gekennzeichnet, dass** der Komplex zusätzlich DNS umfasst, welche für ein Tumorsuppressorprotein kodiert.

13. Komplex nach Anspruch 7, **dadurch gekennzeichnet, dass** das kationische Polymer ausgewählt ist aus der Gruppe bestehend aus Polyethylenimin, vorzugsweise Polycat57, Polylysin, Polyhistidin und Superfect und auch Polyethylenglykol und einen gerichteten Liganden umfassen kann, vorzugsweise das Aminosäurepeptid RGD, Ferritin oder Antikörper, gerichtet gegen HER2.

14. Verwendung nach Anspruch 11, **dadurch gekennzeichnet, dass** das antiangiogene Peptid ausgewählt ist aus der Gruppe bestehend aus: SEQ-ID Nr.: 1, SEQ-ID Nr.: 3, SEQ-ID Nr.: 5, SEQ-ID Nr.: 7, SEQ-ID Nr.: 9, SEQ-ID Nr.: 11, SEQ-ID Nr.: 13, SEQ-ID Nr.: 15, SEQ-ID Nr.: 17, SEQ-ID Nr.: 19, SEQ-ID Nr.: 21, SEQ-ID Nr.: 23, SEQ-ID Nr.: 25, SEQ-ID Nr.: 27 und SEQ-ID Nr.: 30.

15. Verwendung nach Anspruch 11 oder 12, **dadurch gekennzeichnet, dass** die DNS, die für ein antiangiogenes Peptid kodiert, in einer Menge von 0,016 bis 0,33 µg/µg Polymer vorliegt.

16. Verwendung nach Anspruch 11 oder 12, **dadurch gekennzeichnet, dass** die DNS, die für ein Tumorsuppressorprotein kodiert, in einer Menge von 0,016 bis 0,33 µg/µg Polymer vorliegt.

## Revendications

1. Complexe liposome cationique - ADN comprenant un ADN codant pour un peptide anti-angiogenèse, ledit complexe comprend en outre un ADN codant pour une protéine suppresseur de tumeur, dans lequel ladite protéine suppresseur de tumeur est p53 et dans lequel ledit peptide anti-angiogenèse est choisi dans le groupe comprenant la SEQ ID NO : 1, la SEQ ID NO : 3, la SEQ ID NO : 5, la SEQ ID NO : 7, la SEQ ID NO : 9, la SEQ ID NO : 11, la SEQ ID NO : 13, la SEQ ID NO : 15, la SEQ ID NO : 17, la SEQ ID NO : 19, la SEQ ID NO : 21, la SEQ ID NO : 23, la SEQ ID NO : 25, la SEQ ID NO : 27 et la SEQ ID NO : 30.

2. Complexe selon la revendication 1, dans lequel lesdits liposomes cationiques sont composés d'un lipide cationique, de préférence la 1,2-dioléoyl-sn-glycéro-3-éthylphosphocholine, la 1,2-dimyristoyl-sn-glycéro-3-éthylphosphocholine et le chlorure de N-[1-(2,3-dioleoyloxy)-propyl]-N,N,N-triméthylammonium et peuvent également être composés de polyéthylène glycol, de préférence un lipid-1,2-diacyl-sn-glycéro-3-phosphoéthanolamine-N-poly(éthylène glycol) 2000 pégylé et d'un ligand ciblé, de préférence le peptide à acides aminés RGD, la ferritine ou des anticorps dirigés contre HER2.

3. Complexe selon la revendication 1, dans lequel lesdits liposomes cationiques sont composés sur leur membrane externe par des liaisons avec une polyéthylénimine polymère cationique, de préférence Polycat 57, la polylysine, la polyhistidine et Superfect et peuvent également être composés de polyéthylène glycol et d'un ligand 1 ciblé, de préférence le peptide à acides aminés RGD, la ferritine ou des anticorps dirigés contre HER2, CEA.

4. Complexe selon la revendication 1, dans lequel ledit ADN codant pour une protéine suppresseur de tumeur est présent dans une quantité allant de 0,0025 à 0,16 µg/pM de liposome.

5. Complexe selon la revendication 1, dans lequel ledit ADN codant pour une protéine suppresseur de tumeur est présent dans une quantité allant de 0,016 à 0,33 µg/pM de liposome.

6. Utilisation d'un complexe pour la production d'un médicament pour inhiber l'angiogenèse tumorale selon la revendication 1, dans laquelle ledit liposome cationique comprend de préférence la 1,2-dioléoyl-sn-glycéro-3-éthylphosphocholine, la 1,2-dimyristoyl-sn-glycéro-3-éthylphosphocholine et le chlorure de N-[1-(2,3-dioleoyloxy)propyl]-N,N,N-triméthylammonium et peut également être composé de polyéthylène glycol, de préférence un lipid-1,2-diacyl-sn-glycéro-3-phospho-éthanolamine-N-poly(éthylène glycol) 2000 pégylé et d'un ligand ciblé, de préférence le peptide à acides aminés RGD, la ferritine ou des anticorps dirigés contre HER2.

7. Complexe polymère cationique - ADN comprenant l'ADN codant pour un peptide anti-angiogenèse, dans lequel ledit complexe comprend en outre un ADN codant pour une protéine suppresseur de tumeur, dans lequel ladite protéine suppresseur de tumeur est p53 et dans lequel ledit peptide anti-angiogenèse est choisi dans le groupe comprenant la SEQ ID NO : 1, la SEQ ID NO : 3, la SEQ ID NO : 5, la SEQ ID NO : 7, la SEQ ID NO : 9, la SEQ ID NO : 11, la SEQ ID NO : 13, la SEQ ID NO : 15, la SEQ ID NO : 17, la SEQ ID NO : 19, la SEQ ID NO : 21, la SEQ ID NO : 23, la SEQ ID NO : 25, la SEQ ID NO : 27 et la SEQ ID NO : 30.

8. Complexe selon la revendication 7, dans lequel ledit ADN codant pour un peptide anti-angiogenèse est présent dans une quantité allant de 0,016 à 0,33 µg/µg de polymère.

9. Complexe selon la revendication 7, dans lequel ledit ADN codant pour une protéine suppresseur de tumeur est présent dans une quantité allant de 0,0025 à 0,16 µg/pM.

10. Complexe selon la revendication 7, dans lequel ledit ADN codant pour une protéine suppresseur de tumeur est présent dans une quantité allant de 0,016 à 0,33 µg/pM.

11. Utilisation d'un complexe polymère cationique - ADN selon la revendication 7, comprenant un ADN codant pour un peptide anti-angiogenèse pour la production d'un médicament pour inhiber la croissance tumorale chez un sujet, qui comprend de préférence l'administration ce dernier à un sujet porteur de tumeur.

12. Utilisation selon la revendication 11, où ledit complexe comprend en outre un ADN codant pour une protéine suppresseur de tumeur.

13. Complexe selon la revendication 7, dans lequel ledit polymère cationique est choisi dans le groupe comprenant une polyéthylénimine de préférence Polycat 57, la polylysine, la polyhistidine, et Superfect et peut également être composé de polyéthylène glycol et d'un ligand ciblé, de préférence le peptide à acides aminés RGD, la ferritine ou des anticorps dirigés contre HER2.

14. Utilisation selon la revendication 11, dans laquelle ledit peptide anti-angiogenèse est choisi dans le groupe comprenant la SEQ ID NO : 1, la SEQ ID NO : 3, la SEQ ID NO : 5, la SEQ ID NO : 7, la SEQ ID NO : 9, la SEQ ID NO : 11, la SEQ ID NO : 13, la SEQ ID NO : 15, la SEQ ID NO : 17, la SEQ ID NO : 19, la SEQ ID NO : 21, la SEQ ID NO : 23, la SEQ ID NO : 25, la SEQ ID NO : 27 et la SEQ ID NO : 30.

15. Utilisation selon la revendication 11 ou la revendication 12, dans laquelle ledit ADN codant pour un peptide anti-angiogenèse est présent dans une quantité allant de 0,016 à 0,33 µg/µg de polymère.

16. Utilisation selon la revendication 11 ou la revendication 12, dans laquelle ledit ADN codant pour une protéine suppresseur de tumeur est présent dans une quantité allant de 0,016 à 0,33 µg/µg de polymère.


### SEQUENCE LISTING

(1) GENERAL INFORMATION:
   (i) APPLICANT:
      (A) NAME: Archibald James MIXSON
      (B) STREET: 1 Baederwood Ct.
      (C) CITY: Rockville
      (D) STATE: Maryland
      (E) COUNTRY: USA
      (F) POSTAL CODE (ZIP): 21201
   (ii) TITLE OF INVENTION: CATIONIC VEHICLE:DNA COMPLEXES AND THEIR USE IN GENE THERAPY
   (iii) NUMBER OF SEQUENCES: 33
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 97112154.6
(2) INFORMATION FOR SEQ ID NO: 1:
   (i) SEQUENCE CHARACTERISTICS:
      (A) LENGTH: 217 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 1:
   Met Thr Glu Asn Lys Glu Leu Ala Asn Glu Leu Arg Arg Pro Pro Leu
   1               5                   10                  15
   Cys Tyr His Asn Gly Val Gln Tyr Arg Asn Asn Glu Glu Trp Thr Val
               20                  25                  30
   Asp Ser Cys Thr Glu Cys His Cys Gln Asn Ser Val Thr Ile Cys Lys
           35                  40                  45
   Lys Val Ser Cys Pro Ile Met Pro Cys Ser Asn Ala Thr Val Pro Asp
       50                  55                  60
   Glu Glu Cys Cys Pro Arg Cys Trp Pro Ser Asp Ser Ala Asp Asp Gly
   65                  70                  75                  80
   Trp Ser Pro Trp Ser Glu Trp Thr Ser Cys Ser Thr Ser Cys Gly Asn
                   85                  90                  95
   Gly Ile Gln Gln Arg Gly Arg Ser Cys Asp Ser Leu Asn Asn Arg Cys
               100                 105                 110
   Glu Gly Ser Ser Val Gln Thr Arg Thr Cys His Ile Gln Glu Cys Asp
           115                 120                 125
   Lys Arg Phe Lys Gln Asp Gly Gly Trp Ser His Trp Ser Pro Trp Ser
       130                 135                 140
   Ser Cys Val Thr Cys Gly Asp Gly Val Ile Thr Arg Ile Thr Leu Cys
   145                 150                 155                 160
   Asn Ser Pro Ser Pro Gln Met Asn Gly Lys Pro Cys Glu Gly Glu Ala
                   165                 170                 175
   Arg Glu Thr Lys Lys Ala Cys Lys Lys Asp Ala Cys Pro Ile Asn Glu
               180                 185                 190
   Gly Trp Gly Pro Trp Ser Pro Trp Asp Ile Cys Ser Val Thr Cys Gly
           195                 200                 205
   Gly Gly Val Gln Lys Arg Ser Arg Leu
       210                 215
(2) INFORMATION FOR SEQ ID NO: 2:
   (i) SEQUENCE CHARACTERISTICS:
      (A) LENGTH: 656 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 2:
   ATGACTGAAG AGAACAAAGA GTTGGCCAAT GAGCTGAGGC GGCCTCCCCT ATGCTATCAC    60
   AACGGAGTTC AGTACAGAAA TAACGAGGAA TGGACTGTTG ATAGCTGCAC TGAGTGTCAC   120
   TGTCAGAACT CAGTTACCAT CTGCAAAAAG GTGTCCTGCC CCATCATGCC CTGCTCCAAT   180
   GCCACAGTTC CTGATGGAGA ATGCTGTCCT CGCTGTTGGC CCAGCGACTC TGCGGACGAT   240
   GGCTGGTCTC CATGGTCCGA GTGGACCTCC TGTTCTACGA GCTGTGGCAA TGGAATTCAG   300
   CAGCGCGGCC GCTCCTGCGA TAGCCTCAAC AACCGATGTG AGGGCTCCTC GGTCCAGACA   360
   CGGACCTGCC ACATTCAGGA GTGTGACAAA AGATTTAAAC AGGATGGTGG CTGGAGCCAC   420
   TGGTCCCCGT GGTCATCTTG TTCTGTGACA TGTGGTGATG GTGTGATCAC AAGGATCCGG   480
   CTCTGCAACT CTCCCAGCCC CCAGATGAAT GGGAAACCCT GTGAAGGCGA AGCGCGGGAG   540
   ACCAAAGCCT GCAAGAAAGA CGCCTGCCCC ATCAATGGAG GCTGGGGTCC TTGGTCACCA   600
   TGGGACATCT GTTCTGTCAC CTGTGGAGGA GGGGTACAGA AACGTAGTCT CTCTAA       656
(2) INFORMATION FOR SEQ ID NO: 3:
   (i) SEQUENCE CHARACTERISTICS:
      (A) LENGTH: 439 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 3:
   Met Thr Glu Asn Lys Glu Leu Ala Asn Glu Leu Arg Arg Pro Pro Leu
   1               5                   10                  15
   Cys Tyr His Asn Gly Val Gln Tyr Arg Asn Asn Glu Glu Trp Thr Val
               20                  25                  30
   Asp Ser Cys Thr Glu Cys His Cys Gln Asn Ser Val Thr Ile Cys Lys
           35                  40                  45
   Lys Val Ser Cys Pro Ile Met Pro Cys Ser Asn Ala Thr Val Pro Asp
       50                  55                  60
   Glu Glu Cys Cys Pro Arg Cys Trp Pro Ser Asp Ser Ala Asp Asp Gly
   65                  70                  75                  80
   Trp Ser Pro Trp Ser Glu Trp Thr Ser Cys Ser Thr Ser Cys Gly Asn
                   85                  90                  95
   Gly Ile Gln Gln Arg Gly Arg Ser Cys Asp Ser Leu Asn Asn Arg Cys
               100                 105                 110
   Glu Gly Ser Ser Val Gln Thr Arg Thr Cys His Ile Gln Glu Cys Asp
           115                 120                 125
   Lys Arg Phe Lys Gln Asp Gly Gly Trp Ser His Trp Ser Pro Trp Ser
       130                 135                 140
   Ser Cys Val Thr Cys Gly Asp Gly Val Ile Thr Arg Ile Thr Leu Cys
   145                 150                 155                 160
   Asn Ser Pro Ser Pro Gln Met Asn Gly Lys Pro Cys Glu Gly Glu Ala
                   165                 170                 175
   Arg Glu Thr Lys Lys Ala Cys Lys Lys Asp Ala Cys Pro Ile Asn Glu
               180                 185                 190
   Gly Trp Gly Pro Trp Ser Pro Trp Asp Ile Cys Ser Val Thr Cys Gly
           195                 200                 205
   Gly Gly Val Gln Lys Arg Ser Arg Leu Cys Val His Ser Arg Met Thr
       210                 215                 220
   Glu Asn Lys Glu Leu Ala Asn Glu Leu Arg Arg Pro Pro Leu Cys Tyr
   225                 230                 235                 240
   His Asn Gly Val Gln Tyr Arg Asn Asn Glu Glu Trp Thr Val Asp Ser
                   245                 250                 255
   Cys Thr Glu Cys His Cys Gln Asn Ser Val Thr Ile Cys Lys Lys Val
               260                 265                 270
   Ser Cys Pro Ile Met Pro Cys Ser Asn Ala Thr Val Pro Asp Glu Glu
           275                 280                 285
   Cys Cys Pro Arg Cys Trp Pro Ser Asp Ser Ala Asp Asp Gly Trp Ser
       290                 295                 300
   Pro Trp Ser Glu Trp Thr Ser Cys Ser Thr Ser Cys Gly Asn Gly Ile
   305                 310                 315                 320
   Gln Gln Arg Gly Arg Ser Cys Asp Ser Leu Asn Asn Arg Cys Glu Gly
                   325                 330                 335
   Ser Ser Val Gln Thr Arg Thr Cys His Ile Gln Glu Cys Asp Lys Arg
               340                 345                 350
   Phe Lys Gln Asp Gly Gly Trp Ser His Trp Ser Pro Trp Ser Ser Cys
           355                 360                 365
   Val Thr Cys Gly Asp Gly Val Ile Thr Arg Ile Thr Leu Cys Asn Ser
       370                 375                 380
   Pro Ser Pro Gln Met Asn Gly Lys Pro Cys Glu Gly Glu Ala Arg Glu
   385                 390                 395                 400
   Thr Lys Lys Ala Cys Lys Lys Asp Ala Cys Pro Ile Asn Glu Gly Trp
                   405                 410                 415
   Gly Pro Trp Ser Pro Trp Asp Ile Cys Ser Val Thr Cys Gly Gly Gly
               420                 425                 430
   Val Gln Lys Arg Ser Arg Leu
           435
(2) INFORMATION FOR SEQ ID NO: 4:
   (i) SEQUENCE CHARACTERISTICS:
      (A) LENGTH: 1326 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 4:
   ATGACTGAAG AGAACAAAGA GTTGGCCAAT GAGCTGAGGC GGCCTCCCCT ATGCTATCAC     60
   AACGGAGTTC AGTACAGAAA TAACGAGGAA TGGACTGTTG ATAGCTGCAC TGAGTGTCAC    120
   TGTCAGAACT CAGTTACCAT CTGCAAAAAG GTGTCCTGCC CCATCATGCC CTGCTCCAAT    180
   GCCACAGTTC CTGATGGAGA ATGCTGTCCT CGCTGTTGGC CCAGCGACTC TGCGGACGAT    240
   GGCTGGTCTC CATGGTCCGA GTGGACCTCC TGTTCTACGA GCTGTGGCAA TGGAATTCAG    300
   CAGCGCGGCC GCTCCTGCGA TAGCCTCAAC AACCGATGTG AGGGCTCCTC GGTCCAGACA    360
   CGGACCTGCC ACATTCAGGA GTGTGACAAA AGATTTAAAC AGGATGGTGG CTGGAGCCAC    420
   TGGTCCCCGT GGTCATCTTG TTCTGTGACA TGTGGTGATG GTGTGATCAC AAGGATCCGG    480
   CTCTGCAACT CTCCCAGCCC CCAGATGAAT GGGAAACCCT GTGAAGGCGA AGCGCGGGAG    540
   ACCAAAGCCT GCAAGAAAGA CGCCTGCCCC ATCAATGGAG GCTGGGGTCC TTGGTCACCA    600
   TGGGACATCT GTTCTGTCAC CTGTGGAGGA GGGGTACAGA AACGTAGTCG TCTCTGCGTC    660
   GACTCTAGAA TGACTGAAGA GAACAAAGAG TTGGCCAATG AGCTGAGGCG GCCTCCCCTA    720
   TGCTATCACA ACGGAGTTCA GTACAGAAAT AACGAGGAAT GGACTGTTGA TAGCTGCACT    780
   GAGTGTCACT GTCAGAACTC AGTTACCATC TGCAAAAAGG TGTCCTGCCC CATCATGCCC    840
   TGCTCCAATG CCACAGTTCC TGATGGAGAA TGCTGTCCTC GCTGTTGGCC CAGCGACTCT    900
   GCGGACGATG GCTGGTCTCC ATGGTCCGAG TGGACCTCCT GTTCTACGAG CTGTGGCAAT    960
   GGAATTCAGC AGCGCGGCCG CTCCTGCGAT AGCCTCAACA ACCGATGTGA GGGCTCCTCG   1020
   GTCCAGACAC GGACCTGCCA CATTCAGGAG TGTGACAAAA GATTTAAACA GGATGGTGGC   1080
   TGGAGCCACT GGTCCCCGTG GTCATCTTGT TCTGTGACAT GTGGTGATGG TGTGATCACA   1140
   AGGATCCGGC TCTGCAACTC TCCCAGCCCC CAGATGAATG GGAAACCCTG TGAAGGCGAA   1200
   GCGCGGGAGA CCAAAGCCTG CAAGAAAGAC GCCTGCCCCA TCAATGGAGG CTGGGGTCCT   1260
   TGGTCACCAT GGGACATCTG TTCTGTCACC TGTGGAGGAG GGGTACAGAA ACGTAGTCGT   1320
   CTCTAA                                                              1326
(2) INFORMATION FOR SEQ ID NO: 5:
   (i) SEQUENCE CHARACTERISTICS:
      (A) LENGTH: 6 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 5:
   Met Tyr Ile Gly Ser Arg
   1               5
(2) INFORMATION FOR SEQ ID NO: 6:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 6:
      GTCGACATGT ATATTGGTTC TCGTTAAGTC GAC 33
(2) INFORMATION FOR SEQ ID NO: 7:
   (i) SEQUENCE CHARACTERISTICS:
      (A) LENGTH: 25 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 7:
   Met Tyr Ile Gly Ser Arg Gly Lys Ser Tyr Ile Gly Ser Arg Gly Lys
   1               5                   10                  15
   Ser Tyr Ile Gly Ser Arg Gly Lys Ser
               20                  25
(2) INFORMATION FOR SEQ ID NO: 8:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 8:
      GTCGACATGT ATATTGGTTC TCGTGTAAAA GATATATTGG TTCTCGTGGT AAAAGATATA 60
      TTGGTTCTCG TGGTAAAAGA TAAGTCGACC 90
(2) INFORMATION FOR SEQ ID NO: 9:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 9:
   Met Leu Tyr Lys Lys Ile Ile Lys Lys Leu Leu Glu Ser
   1               5                   10
(2) INFORMATION FOR SEQ ID NO: 10:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 10:
      GTCGACATGC TTTATAAGAA GATCATCAAG AAGCTTCTTG AGAGTTAAGT CGAC 54
(2) INFORMATION FOR SEQ ID NO: 11:
   (i) SEQUENCE CHARACTERISTICS:
      (A) LENGTH: 46 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 11:
   Met Leu Tyr Lys Lys Ile Ile Lys Lys Leu Leu Glu Ser Gly Lys Ser
   1               5                   10                  15
   Leu Tyr Lys Lys Ile Ile Lys Lys Leu Leu Glu Ser Gly Lys Ser Leu
               20                  25                  30
   Tyr Lys Lys Ile Ile Lys Lys Leu Leu Glu Ser Gly Lys Ser
           35                  40                  45
(2) INFORMATION FOR SEQ ID NO: 12:
   (i) SEQUENCE CHARACTERISTICS:
      (A) LENGTH: 153 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 12:
   GTCGACATGC TTTATAAGAA GATCATCAAG AAGCTTCTTG AGAGTGGTAA AAGACTTTAT 60
   AAGAAGATCA TCAAGAAGCT TCTTGAGAGT GGTAAAAGAC TTTATAAGAA GATCATCAAG 120
   AAGCTTCTTG AGAGTGGTAA AAGATAAGTC GAC 153
(2) INFORMATION FOR SEQ ID NO: 13:
   (i) SEQUENCE CHARACTERISTICS:
      (A) LENGTH: 9 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 13:
   Met Phe Cys Tyr Trp Lys Val Cys Trp
   1 5
(2) INFORMATION FOR SEQ ID NO: 14:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 14:
      GTCGACATGT TCTTGTATTG GAAGGGATTG TGGTAAGTCG AC 42
(2) INFORMATION FOR SEQ ID NO: 15:
   (i) SEQUENCE CHARACTERISTICS:
      (A) LENGTH: 33 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 15:
   Met Phe Cys Tyr Trp Lys Val Cys Trp Gly Lys Ser Phe Cys Tyr Trp
   1               5                   10                  15
   Lys Val Cys Trp Gly Lys Ser Phe Cys Trp Lys Val Cys Trp Gly Lys
               20                  25                  30
   Ser
(2) INFORMATION FOR SEQ ID NO: 16:
   (i) SEQUENCE CHARACTERISTICS:
      (A) LENGTH: 117 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 16:
      GTCGACATGT TCTTGTATTG GAAGGGATTG TGGGGTAAAA GATTCTTGTA TTGGAAGGGA 60
      TTGTGGGGTA AAAGATTCTT GTATTGGAAG GGATTGTGGG GTAAAAGATA AGTCGAC 117
(2) INFORMATION FOR SEQ ID NO: 17:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 17:
   Met Gly Arg Gly Asp
   1               5
(2) INFORMATION FOR SEQ ID NO: 18:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 18:
      GTCGACATGT CTTTGTCTTG GAAGACTTTG ACTTAAGTCG AC 42
(2) INFORMATION FOR SEQ ID NO: 19:
   (i) SEQUENCE CHARACTERISTICS:
      (A) LENGTh: 22 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 19:
   Met Gly Arg Gly Asp Gly Lys Ser Gly Arg Gly Asp Gly Lys Ser Gly
   1               5                   10                  15
   Arg Gly Asp Gly Lys Ser
               20
(2) INFORMATION FOR SEQ ID NO: 20:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 20:
      GTCGACATGG GTCGTGGTGA TGGTAAAAGA GGTCGTGGTG ATGGTAAAAG AGGTCGTGGT 60
      GATGGTAAAA GATAAGTCGA C 81
(2) INFORMATION FOR SEQ ID NO: 21:
   (i) SEQUENCE CHARACTERISTICS:
      (A) LENGTH: 310 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 21:
   Met Val Tyr Leu Ser Glu Cys Lys Thr Gly Ile Gly Asn Gly Tyr Arg
   1 5 10 15
   Gly Thr Met Ser Arg Thr Lys Ser Gly Val Ala Cys Gln Lys Trp Gly
   20 25 30
   Ala Thr Phe Pro His Val Pro Asn Tyr Ser Pro Ser Thr His Pro Asn
   35 40 45
   Glu Gly Leu Glu Glu Asn Tyr Cys Arg Asn Pro Asp Asn Asp Glu Gln
   50 55 60
   Gly Pro Trp Cys Tyr Thr Thr Asp Pro Asp Lys Arg Tyr Asp Tyr Cys
   65 70 75 80
   Asn Ile Pro Glu Cys Glu Glu Glu Cys Met Tyr Cys Ser Gly Glu Lys
   85 90 95
   Tyr Glu Gly Lys Ile Ser Lys Thr Met Ser Gly Lys Asp Cys Gln Ala
   100 105 110
   Trp Asp Ser Gln Ser Pro His Ala His Gly Tyr Ile Pro Ala Lys Phe
   115 120 125
   Pro Ser Lys Asn Leu Lys Met Asn Tyr Cys His Asn Pro Asp Gly Glu
   130 135 140
   Pro Arg Pro Trp Cys Phe Thr Thr Asp Pro Thr Lys Arg Trp Glu Tyr
   145 150 155 160
   Cys Asp Ile Pro Arg Cys Thr Thr Pro Pro Pro Pro Pro Ser Pro Thr
                   165                 170                 175
   Tyr Gln Cys Leu Lys Gly Arg Gly Glu Asn Tyr Arg Gly Thr Val Ser
               180                 185                 190
   Val Thr Val Ser Gly Lys Thr Cys Gln Arg Trp Ser Glu Gln Thr Pro
           195                 200                 205
   His Arg His Asn Arg Thr Pro Glu Asn Phe Pro Cys Arg Asn Leu Glu
       210                 215                 220
   Glu Asn Tyr Cys Arg Asn Pro Asp Gly Glu Thr Ala Pro Trp Cys Tyr
   225                 230                 235                 240
   Thr Thr Asp Ser Gln Leu Arg Trp Glu Tyr Cys Glu Ile Pro Ser Cys
                   245                 250                 255
   Glu Ser Ser Ala Ser Pro Asp Gln Ser Asp Ser Ser Val Pro Pro Glu
               260                 265                 270
   Glu Gln Thr Pro Val Val Gln Glu Cys Tyr Gln Ser Asp Gly Gln Ser
           275                 280                 285
   Tyr Arg Gly Thr Ser Ser Thr Thr Ile Thr Gly Lys Lys Cys Gln Ser
       290                 295                 300
   Glu Gln Thr Pro His Arg
   305                 310
(2) INFORMATION FOR SEQ ID NO: 22:
   (i) SEQUENCE CHARACTERISTICS:
      (A) LENGTH: 645 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 22:
   GTCGACATGG TGTATCTGTC AGAATGTAAG ACCGGCATCG GCAACGGCTA CAGAGGAACC     60
   ATGTCCAGGA CAAAGAGTGG TGTTGCCTGT CAAAAGTGGG GTGCCACGTT CCCCCACGTA    120
   CCCAACTACT CTCCCAGTAC ACATCCCAAT GAGGGACTAG AAGAGAACTA CTGTAGGAAC    180
   CCAGACAATG ATGAACAAGG GCCTTGGTGC TACACTACAG ATCCGGACAA GAGATATGAC    240
   TACTGCAACA TTCCTGAATG TGAAGAGGAA TGCATGTACT GCAGTGGAGA AAAGTATGAG    300
   GGCAAAATCT CCAAGACCAT GTCTGGACTT GACTGCCAGG CCTGGGATTC TCAGAGCCCA    360
   CATGCTCATG GATACATCCC TGCCAAATTT CCAAGCAAGA ACCTGAAGAT GAATTATTGC    420
   CACAACCCTG ACGGGGAGCC AAGGCCCTGG TGCTTCACAA CAGACCCCAC CAAACGCTGG    480
   GAATACTGTG ACATCCCCCG CTGCACAACA CCCCCGCCCC CACCCAGCCC AACCTACCAA    540
   TGTCTGAAAG GAAGAGGTGA AAATTACCGA GGGACCGTGT CTGTCACCGT GTCTGGGAAA    600
   ACCTGTCAGC GCTGGAGTGA GCAAACCCCT CATAGGTGAG TCGAC                    645
(2) INFORMATION FOR SEQ ID NO: 23:
   (i) SEQUENCE CHARACTERISTICS:
      (A) LENGTH: 623 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 23:
   Met Val Tyr Leu Ser Glu Cys Lys Thr Gly Ile Gly Asn Gly Tyr Arg
   1 5 10 15
   Gly Thr Met Ser Arg Thr Lys Ser Gly Val Ala Cys Gln Lys Trp Gly
   20 25 30
   Ala Thr Phe Pro His Val Pro Asn Tyr Ser Pro Ser Thr His Pro Asn
   35 40 45
   Glu Gly Leu Glu Glu Asn Tyr Cys Arg Asn Pro Asp Asn Asp Glu Gln
   50 55 60
   Gly Pro Trp Cys Tyr Thr Thr Asp Pro Asp Lys Arg Tyr Asp Tyr Cys
   65 70 75 80
   Asn Ile Pro Glu Cys Glu Glu Glu Cys Met Tyr Cys Ser Gly Glu Lys
   85 90 95
   Tyr Glu Gly Lys Ile Ser Lys Thr Met Ser Gly Lys Asp Cys Gln Ala
   100 105 110
   Trp Asp Ser Gln Ser Pro His Ala His Gly Tyr Ile Pro Ala Lys Phe
   115 120 125
   Pro Ser Lys Asn Leu Lys Met Asn Tyr Cys His Asn Pro Asp Gly Glu
   130 135 140
   Pro Arg Pro Trp Cys Phe Thr Thr Asp Pro Thr Lys Arg Trp Glu Tyr
   145 150 155 160
   Cys Asp Ile Pro Arg Cys Thr Thr Pro Pro Pro Pro Pro Ser Pro Thr
   165 170 175
   Tyr Gln Cys Leu Lys Gly Arg Gly Glu Asn Tyr Arg Gly Thr Val Ser
   180 185 190
   Val Thr Val Ser Gly Lys Thr Cys Gln Arg Trp Ser Glu Gln Thr Pro
   195 200 205
   His Arg His Asn Arg Thr Pro Glu Asn Phe Pro Cys Arg Asn Leu Glu
   210 215 220
   Glu Asn Tyr Cys Arg Asn Pro Asp Gly Glu Thr Ala Pro Trp Cys Tyr
   225 230 235 240
   Thr Thr Asp Ser Gln Leu Arg Trp Glu Tyr Cys Glu Ile Pro Ser Cys
   245 250 255
   Glu Ser Ser Ala Ser Pro Asp Gln Ser Asp Ser Ser Val Pro Pro Glu
   260 265 270
   Glu Gln Thr Pro Val Val Gln Glu Cys Tyr Gln Ser Asp Gly Gln Ser
   275 280 285
   Tyr Arg Gly Thr Ser Ser Thr Thr Ile Thr Gly Lys Lys Cys Gln Ser
   290 295 300
   Glu Gln Thr Pro His Arg Gly Lys Ser Met Val Tyr Leu Ser Glu Cys
   305 310 315 320
   Lys Thr Gly Ile Gly Asn Gly Tyr Arg Gly Thr Met Ser Arg Thr Lys
   325 330 335
   Ser Gly Val Ala Cys Gln Lys Trp Gly Ala Thr Phe Pro His Val Pro
   340 345 350
   Asn Tyr Ser Pro Ser Thr His Pro Asn Glu Gly Leu Glu Glu Asn Tyr
   355 360 365
   Cys Arg Asn Pro Asp Asn Asp Glu Gln Gly Pro Trp Cys Tyr Thr Thr
   370 375 380
   Asp Pro Asp Lys Arg Tyr Asp Tyr Cys Asn Ile Pro Glu Cys Glu Glu
   385 390 395 400
   Glu Cys Met Tyr Cys Ser Gly Glu Lys Tyr Glu Gly Lys Ile Ser Lys
   405 410 415
   Thr Met Ser Gly Lys Asp Cys Gln Ala Trp Asp Ser Gln Ser Pro His
   420 425 430
   Ala His Gly Tyr Ile Pro Ala Lys Phe Pro Ser Lys Asn Leu Lys Met
   435 440 445
   Asn Tyr Cys His Asn Pro Asp Gly Glu Pro Arg Pro Trp Cys Phe Thr
   450 455 460
   Thr Asp Pro Thr Lys Arg Trp Glu Tyr Cys Asp Ile Pro Arg Cys Thr
   465 470 475 480
   Thr Pro Pro Pro Pro Pro Ser Pro Thr Tyr Gln Cys Leu Lys Gly Arg
   485 490 495
   Gly Glu Asn Tyr Arg Gly Thr Val Ser Val Thr Val Ser Gly Lys Thr
   500 505 510
   Cys Gln Arg Trp Ser Glu Gln Thr Pro His Arg His Asn Arg Thr Pro
   515 520 525
   Glu Asn Phe Pro Cys Arg Asn Leu Glu Glu Asn Tyr Cys Arg Asn Pro
   530 535 540
   Asp Gly Glu Thr Ala Pro Trp Cys Tyr Thr Thr Asp Ser Gln Leu Arg
   545 550 555 560
   Trp Glu Tyr Cys Glu Ile Pro Ser Cys Glu Ser Ser Ala Ser Pro Asp
   565 570 575
   Gln Ser Asp Ser Ser Val Pro Pro Glu Glu Gln Thr Pro Val Val Gln
   580 585 590
   Glu Cys Tyr Gln Ser Asp Gly Gln Ser Tyr Arg Gly Thr Ser Ser Thr
   595 600 605
   Thr Ile Thr Gly Lys Lys Cys Gln Ser Glu Gln Thr Pro His Arg
   610 615 620
(2) INFORMATION FOR SEQ ID NO: 24:
   (i) SEQUENCE CHARACTERISTICS:
      (A) LENGTH: 1284 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 24:
   GTCGACATGG TGTATCTGTC AGAATGTAAG ACCGGCATCG GCAACGGCTA CAGAGGAACC 60
   ATGTCCAGGA CAAAGAGTGG TGTTGCCTGT CAAAAGTGGG GTGCCACGTT CCCCCACGTA 120
   CCCAACTACT CTCCCAGTAC ACATCCCAAT GAGGGACTAG AAGAGAACTA CTGTAGGAAC 180
   CCAGACAATG ATGAACAAGG GCCTTGGTGC TACACTACAG ATCCGGACAA GAGATATGAC 240
   TACTGCAACA TTCCTGAATG TGAAGAGGAA TGCATGTACT GCAGTGGAGA AAAGTATGAG 300
   GGCAAAATCT CCAAGACCAT GTCTGGACTT GACTGCCAGG CCTGGGATTC TCAGAGCCCA 360
   CATGCTCATG GATACATCCC TGCCAAATTT CCAAGCAAGA ACCTGAAGAT GAATTATTGC     420
   CACAACCCTG ACGGGGAGCC AAGGCCCTGG TGCTTCACAA CAGACCCCAC CAAACGCTGG     480
   GAATACTGTG ACATCCCCCG CTGCACAACA CCCCCGCCCC CACCCAGCCC AACCTACCAA     540
   TGTCTGAAAG GAAGAGGTGA AAATTACCGA GGGACCGTGT CTGTCACCGT GTCTGGGAAA     600
   ACCTGTCAGC GCTGGAGTGA GCAAACCCCT CATAGGGGTA AAAGAATGGT GTATCTGTCA     660
   GAATGTAAGA CCGGCATCGG CAACGGCTAC AGAGGAACCA TGTCCAGGAC AAAGAGTGGT     720
   GTTGCCTGTC AAAAGTGGGG TGCCACGTTC CCCCACGTAC CCAACTACTC TCCCAGTACA     780
   CATCCCAATG AGGGACTAGA AGAGAACTAC TGTAGGAACC CAGACAATGA TGAACAAGGG     840
   CCTTGGTGCT ACACTACAGA TCCGGACAAG AGATATGACT ACTGCAACAT TCCTGAATGT     900
   GAAGAGGAAT GCATGTACTG CAGTGGAGAA AAGTATGAGG GCAAAATCTC CAAGACCATG     960
   TCTGGACTTG ACTGCCAGGC CTGGGATTCT CAGAGCCCAC ATGCTCATGG ATACATCCCT    1020
   GCCAAATTTC CAAGCAAGAA CCTGAAGATG AATTATTGCC ACAACCCTGA CGGGGAGCCA    1080
   AGGCCCTGGT GCTTCACAAC AGACCCCACC AAACGCTGGG AATACTGTGA CATCCCCCGC    1140
   TGCACAACAC CCCCGCCCCC ACCCAGCCCA ACCTACCAAT GTCTGAAAGG AAGAGGTGAA    1200
   AATTACCGAG GGACCGTGTC TGTCACCGTG TCTGGGAAAA CCTGTCAGCG CTGGAGTGAG    1260
   CAAACCCCTC ATAGGTGAGT CGAC                                           1284
(2) INFORMATION FOR SEQ ID NO: 25:
   (i) SEQUENCE CHARACTERISTICS:
      (A) LENGTH: 125 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 25:
   Met Leu Pro Ile Cys Pro Gly Gly Ala Ala Arg Cys Gln Val Thr Leu
   1               5                   10                  15
   Arg Glu Leu Phe Asp Arg Ala Val Val Leu Ser His Tyr Ile His Asn
               20                  25                  30
   Leu Ser Ser Glu Met Phe Ser Glu Phe Glu Lys Arg Tyr Thr His Gly
           35                  40                  45
   Arg Gly Phe Ile Thr Lys Ala Ile Asn Ser Cys His Thr Ser Ser Leu
       50                  55                  60
   Ala Thr Pro Glu Asp Lys Glu Gln Ala Gln Gln Met Asn Gln Lys Asp
   65                  70                  75                  80
   Phe Leu Ser Leu Ile Val Ser Ile Leu Arg Ser Trp Asn Glu Pro Leu
                   85                  90                  95
   Tyr His Leu Val Thr Glu Val Arg Gly Met Gln Glu Ala Pro Gln Ala
               100                 105                 110
   Ile Leu Ser Lys Ala Val Glu Ile Glu Glu Gln Thr Lys
           115                 120                 125
(2) INFORMATION FOR SEQ ID NO: 26:
   (i) SEQUENCE CHARACTERISTICS:
      (A) LENGTH: 387 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 26:
   GTCGACATGT TGCCCATCTG TCCCGGCGGG GCTGCCCGAT GCCAGGTGAC CCTTCGAGAC      60
   CTGTTTGACC GCGCCGTCGT CCTGTCCCAC TACATCCATA ACCTCTCCTC AGAAATGTTC     120
   AGCGAATTCG ATAAACGGTA TACCCATGGC CGGGGGTTCA TTACCAAGGC CATCAACAGC     180
   TGCCACACTT CTTCCCTTGC CACCCCCGAA GACAAGGAGC AAGCCCAACA GATGAATCAA     240
   AAAGACTTTC TGAGCCTGAT AGTCAGCATA TTGCGATCCT GGAATGAGCC TCTGTATCAT     300
   CTGGTCACGG AAGTACGTGG TATGCAAGAA GCCCCGGAGG CTATCCTATC CAAAGCTGTA     360
   GAGATTGAGG AGCAAACCTA AGTCGAC                                         387
(2) INFORMATION FOR SEQ ID NO: 27:
   (i) SEQUENCE CHARACTERISTICS:
      (A) LENGTH: 255 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 27:
(2) INFORMATION FOR SEQ ID NO: 28:
   (i) SEQUENCE CHARACTERISTICS:
      (A) LENGTH: 756 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 28:
   ATGTTGCCCA TCTGTCCCGG CGGGGCTGCC CGATGCCAGG TGACCCTTCG AGACCTGTTT     60
   GACCGCGCCG TCGTCCTGTC CCACTACATC CATAACCTCT CCTCAGAAAT GTTCAGCGAA    120
   TTCGATAAAC GGTATACCCA TGGCCGGGGG TTCATTACCA AGGCCATCAA CAGCTGCCAC    180
   ACTTCTTCCC TTGCCACCCC CGAAGACAAG GAGCAAGCCC AACAGATGAA TCAAAAAGAC    240
   TTTCTGAGCC TGATAGTCAG CATATTGCGA TCCTGGAATG AGCCTCTGTA TCATCTGGTC    300
   ACGGAAGTAC GTGGTATGCA AGAAGCCCCG GAGGCTATCC TATCCAAAGC TGTAGAGATT    360
   GAGGAGCAAA CCGGTAAAAG AATGTTGCCC ATCTGTCCCG GCGGGGCTGC CCGATGCCAG    420
   GTGACCCTTC GAGACCTGTT TGACCGCGCC GTCGTCCTGT CCCACTACAT CCATAACCTC    480
   TCCTCAGAAA TGTTCAGCGA ATTCGATAAA CGGTATACCC ATGGCCGGGG GTTCATTACC    540
   AAGGCCATCA ACAGCTGCCA CACTTCTTCC CTTGCCACCC CCGAAGACAA GGAGCAAGCC    600
   CAACAGATGA ATCAAAAAGA CTTTCTGAGC CTGATAGTCA GCATATTGCG ATCCTGGAAT    660
   GAGCCTCTGT ATCATCTGGT CACGGAAGTA CGTGGTATGC AAGAAGCCCC GGAGGCTATC    720
   CTATCCAAAG CTGTAGAGAT TGAGGAGCAA ACCTAA                              756
(2) INFORMATION FOR SEQ ID NO: 23:
   (i) SEQUENCE CHARACTERISTICS:
      (A) LENGTH: 161 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 29:
(2) INFORMATION FOR SEQ ID NO: 30:
   (i) SEQUENCE CHARACTERISTICS:
      (A) LENGTH: 63 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 30:
   Met Leu Arg Arg Pro Pro Leu Cys Tyr His Asn Gly Val Gln Tyr Arg
   1               5                   10                  15
   Asn Asn Glu Glu Trp Thr Val Asp Ser Gly Lys Ser Ser Pro Trp Ser
               20                  25                  30
   Ser Cys Ser Val Thr Cys Gly Asp Gly Val Ile Thr Arg Ile Gly Lys
           35                  40                  45
   Ser Ser Pro Trp Asp Ile Cys Ser Val Thr Cys Gly Gly Gly Val
       50                  55                  60
(2) INFORMATION FOR SEQ ID NO: 31:
   (i) SEQUENCE CHARACTERISTICS:
      (A) LENGTH: 185 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 31:
   ATGCTGAGGC GGCCTCCCCT ATGCTATCAC AACGGAGTTC AGTACAGAAA TAACGGTAAA     60
   AGATCCCCGT GGTCATCTTG TTCTGTGACA TGTGGTGATG GTGTGATGGT AAAAGAAGTG    120
   GTACCCTGTA GACAAGACAG TGGACACCTC CTCCCCATTA TATTGGTTCT CGTGGTAAAA    180
   GATAA                                                                185
(2) INFORMATION FOR SEQ ID NO: 32:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 32:
      TAGGTCTAGA ATGACTGAAG AGAACAAAGA G 31
(2) INFORMATION FOR SEQ ID NO: 33:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 33:
      ATGGTCTAGA TTAGAGACGA CTACGTTTCT G 31

### SEQUENCE LISTING

(1) GENERAL INFORMATION:
   (i) APPLICANT: Archibald James MIXSON
   (ii) TITLE OF INVENTION: CATIONIC VEHICLE:DNA COMPLEXES AND THEIR USE IN GENE THERAPY
   (iii) NUMBER OF SEQUENCES: 25
   (iv) CORRESPONDENCE ADDRESS:
      (A) ADDRESSEE: COHAUSZ HASE DAWIDOWICZ
      (B) STREET: Schumannstr. 97-99
      (C) CITY: Dusseldorf
      (D) STATE:
      (E) COUNTRY: GERMANY
      (F) ZIP: 40237
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE: July 16,1997
      (C) CLASSIFICATION:
   (viii) ATTORNEY/AGENT INFORMATION:
      (A) NAME: SIECKAMNN, RALF, Dr.
      (B) REGISTRATION NUMBER:
      (C) REFERENCE/DOCKET NUMBER: 570414 (S/uc)
   (ix) TELECOMMUNICATION INFORMATION:
      (A) TELEPHONE: +49/2 11/9 14 60-0
      (B) TELEFAX: +49/2 11/9 14 60-60
**(2) INFORMATION FOR SEQ ID NO:1:**
   (i) SEQUENCE CHARACTERISTICS:
      (A) LENGTH: 207 amino acids
      (B) TYPE: amino acid
      (D) TOPOLOGY: linear
   (ii) MOLECULE TYPE: protein
   (xi) SEQUENCE DESCRIPTION: SEQ ID NO:1:
**(2) INFORMATION FOR SEQ ID NO:2:**
   (i) SEQUENCE CHARACTERISTICS:
      (A) LENGTH: 656 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:2:
   ATGACTGAAG AGAACAAAGA GTTGGCCAAT GAGCTGAGGC GGCCTCCCCT      50
   ATGCTATCAC AACGGAGTTC AGTACAGAAA TAACGAGGAA TGGACTGTTG     100
   ATAGCTGCAC TGAGTGTCAC TGTCAGAACT CAGTTACCAT CTGCAAAAAG     150
   GTGTCCTGCC CCATCATGCC CTGCTCCAAT GCCACAGTTC CTGATGGAGA     200
   ATGCTGTCCT CGCTGTTGGC CCAGCGACTC TGCGGACGAT GGCTGGTCTC     250
   CATGGTCCGA GTGGACCTCC TGTTCTACGA GCTGTGGCAA TGGAATTCAG     300
   CAGCGCGGCC GCTCCTGCGA TAGCCTCAAC AACCGATGTG AGGGCTCCTC     350
   GGTCCAGACA CGGACCTGCC ACATTCAGGA GTGTGACAAA AGATTTAAAC     400
   AGGATGGTGG CTGGAGCCAC TGGTCCCCGT GGTCATCTTG TTCTGTGACA     450
   TGTGGTGATG GTGTGATCAC AAGGATCCGG CTCTGCAACT CTCCCAGCCC     500
   CCAGATGAAT GGGAAACCCT GTGAAGGCGA AGCGCGGGAG ACCAAAGCCT     550
   GCAAGAAAGA CGCCTGCCCC ATCAATGGAG GCTGGGGTCC TTGGTCACCA     600
   TGGGACATCT GTTCTGTCAC CTGTGGAGGA GGGGTACAGA AACGTAGTCT     650
   CTCTAA                                                     656
**(2) INFORMATION FOR SEQ ID NO:3:**
   (i) SEQUENCE CHARACTERISTICS:
      (A) LENGTH: 434 amino acids
      (B) TYPE: amino acid
      (D) TOPOLOGY: linear
   (ii) MOLECULE TYPE: protein
   (xi) SEQUENCE DESCRIPTION: SEQ ID NO:3:
**(2) INFORMATION FOR SEQ ID NO:4:**
   (i) SEQUENCE CHARACTERISTICS:
      (A) LENGTH: 1326 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:4:
   ATGACTGAAG AGAACAAAGA GTTGGCCAAT GAGCTGAGGC GGCCTCCCCT     50
   ATGCTATCAC AACGGAGTTC AGTACAGAAA TAACGAGGAA TGGACTGTTG    100
   ATAGCTGCAC TGAGTGTCAC TGTCAGAACT CAGTTACCAT CTGCAAAAAG    150
   GTGTCCTGCC CCATCATGCC CTGCTCCAAT GCCACAGTTC CTGATGGAGA    200
   ATGCTGTCCT CGCTGTTGGC CCAGCGACTC TGCGGACGAT GGCTGGTCTC    250
   CATGGTCCGA GTGGACCTCC TGTTCTACGA GCTGTGGCAA TGGAATTCAG    300
   CAGCGCGGCC GCTCCTGCGA TAGCCTCAAC AACCGATGTG AGGGCTCCTC      350
   GGTCCAGACA CGGACCTGCC ACATTCAGGA GTGTGACAAA AGATTTAAAC      400
   AGGATGGTGG CTGGAGCCAC TGGTCCCCGT GGTCATCTTG TTCTGTGACA      450
   TGTGGTGATG GTGTGATCAC AAGGATCCGG CTCTGCAACT CTCCCAGCCC      500
   CCAGATGAAT GGGAAACCCT GTGAAGGCGA AGCGCGGGAG ACCAAAGCCT      550
   GCAAGAAAGA CGCCTGCCCC ATCAATGGAG GCTGGGGTCC TTGGTCACCA      600
   TGGGACATCT GTTCTGTCAC CTGTGGAGGA GGGGTACAGA AACGTAGTCG      650
   TCTCTGCGTC GACTCTAGAA TGACTGAAGA GAACAAAGAG TTGGCCAATG      700
   AGCTGAGGCG GCCTCCCCTA TGCTATCACA ACGGAGTTCA GTACAGAAAT      750
   AACGAGGAAT GGACTGTTGA TAGCTGCACT GAGTGTCACT GTCAGAACTC      800
   AGTTACCATC TGCAAAAAGG TGTCCTGCCC CATCATGCCC TGCTCCAATG      850
   CCACAGTTCC TGATGGAGAA TGCTGTCCTC GCTGTTGGCC CAGCGACTCT      900
   GCGGACGATG GCTGGTCTCC ATGGTCCGAG TGGACCTCCT GTTCTACGAG      950
   CTGTGGCAAT GGAATTCAGC AGCGCGGCCG CTCCTGCGAT AGCCTCAACA      1000
   ACCGATGTGA GGGCTCCTCG GTCCAGACAC GGACCTGCCA CATTCAGGAG      1050
   TGTGACAAAA GATTTAAACA GGATGGTGGC TGGAGCCACT GGTCCCCGTG      1100
   GTCATCTTGT TCTGTGACAT GTGGTGATGG TGTGATCACA AGGATCCGGC      1150
   TCTGCAACTC TCCCAGCCCC CAGATGAATG GGAAACCCTG TGAAGGCGAA      1200
   GCGCGGGAGA CCAAAGCCTG CAAGAAAGAC GCCTGCCCCA TCAATGGAGG      1250
   CTGGGGTCCT TGGTCACCAT GGGACATCTG TTCTGTCACC TGTGGAGGAG      1300
   GGGTACAGAA ACGTAGTCGT CTCTAA                                1326
**(2) INFORMATION FOR SEQ ID NO:5:**
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Tyr Ile Gly Ser Arg                                     6
    1               5
**(2) INFORMATION FOR SEQ ID NO:6:**
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:6:
      GTCGACATGT ATATTGGTTC TCGTTAAGTC GAC 33
**(2) INFORMATION FOR SEQ ID NO:7:**
   (i) SEQUENCE CHARACTERISTICS:
      (A) LENGTH: 25 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Tyr Ile Gly Ser Arg Gly Lys Ser Tyr Ile Gly Ser Arg
     1             5                  10
   Gly Lys Ser Tyr Ile Gly Ser Arg Gly Lys Ser
    15                 20                  25
**(2) INFORMATION FOR SEQ ID NO:8:**
   (i) SEQUENCE CHARACTERISTICS:
      (A) LENGTH: 91 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:8:
      GTCGACATGT ATATTGGTTC TCGTGTAAAA GATATATTGG TTCTCGTGG 50
      TAAAAGATAT ATTGGTTCTC GTGGTAAAAG ATAAGTCGAC C 91
**(2) INFORMATION FOR SEQ ID NO:9:**
   (i) SEQUENCE CHARACTERISTICS:
      (A) LENGTH: 13 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Met Leu Tyr Lys Lys Ile Ile Lys Lys Leu Leu Glu Ser
    1               5                   10
**(2) INFORMATION FOR SEQ ID NO:10:**
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:10:
      GTCGACATGC TTTATAAGAA GATCATCAAG AAGCTTCTTG AGAGTTAAGT 50
      CGAC 54
**(2) INFORMATION FOR SEQ ID NO:11:**
   (i) SEQUENCE CHARACTERISTICS:
      (A) LENGTH: 46 amino acids
      (B) TYPE: amino acids
      (D) TOPOLOGY: linear
   (ii) MOLECULE TYPE: protein
   (xi) SEQUENCE DESCRIPTION: SEQ ID NO:11:
   Met Leu Tyr Lys Lys Ile Ile Lys Lys Leu Leu Glu Ser Gly Lys Ser
   1               5                   10                  15
   Leu Tyr Lys Lys Ile Ile Lys Lys Leu Leu Glu Ser Gly Lys Ser Leu
               20                  25                  30
   Tyr Lys Lys Ile Ile Lys Lys Leu Leu Glu Ser Gly Lys Ser
           35                  40                      45
**(2) INFORMATION FOR SEQ ID NO:12:**
   (i) SEQUENCE CHARACTERISTICS:
      (A) LENGTH: 153 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:12:
   GTCGACATGC TTTATAAGAA GATCATCAAG AAGCTTCTTG AGAGTGGTAA     50
   AAGACTTTAT AAGAAGATCA TCAAGAAGCT TCTTGAGAGT GGTAAAAGAC    100
   TTTATAAGAA GATCATCAAG AAGCTTCTTG AGAGTGGTAA AAGATAAGTC    150
   GAC                                                       153
**(2) INFORMATION FOR SEQ ID NO:13:**
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Phe Cys Tyr Trp Lys Val Cys Trp
    1           5
**(2) INFORMATION FOR SEQ ID NO:14:**
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:14:
      GTCGACATGT TCTTGTATTG GAAGGGATTG TGGTAAGTCG AC 42
**(2) INFORMATION FOR SEQ ID NO:15:**
   (i) SEQUENCE CHARACTERISTICS:
      (A) LENGTH: 33 amino acids
      (B) TYPE: amino acids
      (D) TOPOLOGY: linear
   (ii) MOLECULE TYPE: protein
   (xi) SEQUENCE DESCRIPTION: SEQ ID NO:15:
   Met Phe Cys Tyr Trp Lys Val Cys Trp Gly Lys Ser Phe Cys Tyr Trp
   1               5                   10                  15
   Lys Val Cys Trp Gly Lys Ser Phe Cys Trp Lys Val Cys Trp Gly Lys
               20                  25                  30
   Ser
**(2) INFORMATION FOR SEQ ID NO:16:**
   (i) SEQUENCE CHARACTERISTICS:
      (A) LENGTH: 117 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:16:
   GTCGACATGT TCTTGTATTG GAAGGGATTG TGGGGTAAAA GATTCTTGTA     50
   TTGGAAGGGA TTGTGGGGTA AAAGATTCTT GTATTGGAAG GGATTGTGGG     100
   GTAAAAGATA AGTCGAC                                         117
**(2) INFORMATION FOR SEQ ID NO:17:**
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Gly Arg Gly Asp
    1               5
**(2) INFORMATION FOR SEQ ID NO:18:**
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:18:
      GTCGACATGT CTTTGTCTTG GAAGACTTTG ACTTAAGTCG AC 42
**(2) INFORMATION FOR SEQ ID NO:19:**
   (i) SEQUENCE CHARACTERISTICS:
      (A) LENGTH: 22 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Met Gly Arg Gly Asp Gly Lys Ser Gly Arg Gly Asp Gly Lys Ser Gly
    1               5                  10                  15
   Arg Gly Asp Gly Lys Ser
               20
**(2) INFORMATION FOR SEQ ID NO:20:**
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:20:
      GTCGACATGG GTCGTGGTGA TGGTAAAAGA GGTCGTGGTG ATGGTAAAAG 50
      AGGTCGTGGT GATGGTAAAA GATAAGTCGA C 81
**(2) INFORMATION FOR SEQ ID NO:21:**
   i) SEQUENCE CHARACTERISTICS:
      (A) LENGTH: 310 amino acids
      (B) TYPE: amino acid
      (D) TOPOLOGY: linear
   (ii) MOLECULE TYPE: protein
   (xi) SEQUENCE DESCRIPTION: SEQ ID NO:21:
**(2) INFORMATION FOR SEQ ID NO:22:**
   (i) SEQUENCE CHARACTERISTICS:
      (A) LENGTH: 945 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
**(2) INFORMATION FOR SEQ ID NO:23:**
   i) SEQUENCE CHARACTERISTICS:
      (A) LENGTH: 623 amino acids
      (B) TYPE: amino acid
      (D) TOPOLOGY: linear
   (ii) MOLECULE TYPE: protein
   (xi) SEQUENCE DESCRIPTION: SEQ ID NO:23:
**(2) INFORMATION FOR SEQ ID NO:24:**
   (i) SEQUENCE CHARACTERISTICS:
      (A) LENGTH: 1844 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
**(2) INFORMATION FOR SEQ ID NO:25:**
   (i) SEQUENCE CHARACTERISTICS:
      (A) LENGTH: 125 amino acids
      (B) TYPE: amino acids
      (D) TOPOLOGY: linear
   (ii) MOLECULE TYPE: protein
   (xi) SEQUENCE DESCRIPTION: SEQ ID NO:25:
**(2) INFORMATION FOR SEQ ID NO:26:**
   (i) SEQUENCE CHARACTERISTICS:
      (A) LENGTH: 390 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:26:
**(2) INFORMATION FOR SEQ ID NO:27:**
   (i) SEQUENCE CHARACTERISTICS:
      (A) LENGTH: 253 amino acids
      (B) TYPE: amino acids
      (D) TOPOLOGY: linear
   (ii) MOLECULE TYPE: protein
   (xi) SEQUENCE DESCRIPTION: SEQ ID NO:27:
**(2) INFORMATION FOR SEQ ID NO:28:**
   (i) SEQUENCE CHARACTERISTICS:
      (A) LENGTH: 774 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:28:
**(2) INFORMATION FOR SEQ ID NO:29:**
   (i) SEQUENCE CHARACTERISTICS:
      (A) LENGTH: 161 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:29:
   ATGCTGAGGC GGCCTCCCCT ATGCTATCAC AACGGAGTTC AGTACAGAAA    50
   TAACGGTAAA AGATCCCCGT GGTCATCTTG TTCTGTGACA TGTGGTGATG    100
   GTGTGATGGT AAAAGAAGTG GTACCCTGTA GACAAGACAG TGGACACCTC    150
   CTCCCCATTA A                                              161
**(2) INFORMATION FOR SEQ ID NO:30:**
   (i) SEQUENCE CHARACTERISTICS:
      (A) LENGTH: 63 amino acids
      (B) TYPE: amino acid
      (D) TOPOLOGY: linear
   (ii) MOLECULE TYPE: protein
   (xi) SEQUENCE DESCRIPTION: SEQ ID NO:30:
   Met Leu Arg Arg Pro Pro Leu Cys Tyr His Asn Gly Val Gln Tyr Arg Asn
   1               5                   10                  15
   Asn Glu Glu Trp Thr Val Asp Ser Gly Lys Ser Ser Pro Trp Ser Ser Cys
                   20                  25                  30
   Ser Val Thr Cys Gly Asp Gly Val Ile Thr Arg Ile Gly Lys Ser Ser Pro
   35                  40                  45                  50
   Trp Asp Ile Cys Ser Val Thr Cys Gly Gly Gly Val
               55                  60
**(2) INFORMATION FOR SEQ ID NO:31:**
   (i) SEQUENCE CHARACTERISTICS:
      (A) LENGTH: 185 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:31:
   ATGCTGAGGC GGCCTCCCCT ATGCTATCAC AACGGAGTTC AGTACAGAAA 50
   TAACGGTAAA AGATCCCCGT GGTCATCTTG TTCTGTGACA TGTGGTGATG 100
   GTGTGATGGT AAAAGAAGTG GTACCCTGTA GACAAGACAG TGGACACCTC 150
   CTCCCCATTA TATTGGTTCT CGTGGTAAAA GATAA 185
**(2) INFORMATION FOR SEQ ID NO:32:**
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:32:
      TAGGTCTAGA ATGACTGAAG AGAACAAAGA G 31
**(2) INFORMATION FOR SEQ ID NO:33:**
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (xi) SEQUENCE DESCRIPTION: SEQ ID NO:33:
      ATGGTCTAGA TTAGAGACGA CTACGTTTCT G 31